# EUROPEAN PATENT SPECIFICATION

(11) **EP 1 621 597 B1**
(45) Date of publication and mention of the grant of the patent: **18.09.2013**
(21) Application number: 03721011.9
(22) Date of filing: 01.05.2003
(51) Int. Cl.: C09K 11/06, C07C 15/38, H01L 51/50

(54) **1,3,6,8-TETRASUBSTITUTED PYRENE COMPOUNDS, ORGANIC EL DEVICE AND ORGANIC EL DISPLAY**
1,3,6,8-TETRASUBSTITUIERTE PYREN-VERBINDUNGEN, ORGANISCHES EL-GERÄT UND ORGANISCHE EL-ANZEIGE
COMPOSES DE PYRENE 1,3,6,8-TETRASUBSTITUE, DISPOSITIF ET AFFICHAGE ELECTROLUMINESCENTS ORGANIQUES

(43) Date of publication of application: 01.02.2006
(73) Proprietor: UDC Ireland Limited, Dublin 4 (IE)
(72) Inventor: SOTOYAMA, Wataru, c/o FUJITSU LIMITED, Kawasaki-shi, Kanagawa 211-8588 (JP); SATO, Hiroyuki, c/o FUJITSU LIMITED, Kawasaki-shi, Kanagawa 211-8588 (JP); MATSUURA, Azuma, c/o FUJITSU LIMITED, Kawasaki-shi, Kanagawa 211-8588 (JP); KINOSHITA, Masaru, c/o FUJITSU LIMITED, Kawasaki-shi, Kanagawa 211-8588 (JP); TAKAHASHI, Toshiro, c/o FUJITSU LIMITED, Kawasaki-shi, Kanagawa 211-8588 (JP)
(74) Representative: HOFFMANN EITLE
(86) International application number: PCT/JP2003/005577
(87) International publication number: WO 2004/096945

(56) References cited:
- EP-A1- 1 289 343
- EP-A1- 1 403 354
- EP-A2- 0 468 439
- EP-A2- 0 554 569
- EP-A2- 0 564 224
- JP-A- 10 088 122
- JP-A- 2001 118 682
- JP-A- 2002 063 988
- JP-A- 2003 092 186
- US-B1- 6 312 644
- MODRAKOWSKI CLAUDIA ET AL.: 'Synthesis of pyrene containing building blocks for dendrimer synthesis' SYNTHESIS no. 14, 2001, pages 2143 - 2155, XP002970491

## Description

### Technical Field

The present invention relates to 1,3,6,8-tetrasubstituted pyrene compounds suited for light emitting materials in organic electroluminescent elements (hereinafter referring to as "organic EL elements"), organic EL elements comprising the 1,3,6,8-tetrasubstituted pyrene compound, and organic EL displays comprising the organic EL element.

### Background Art

Organic EL elements may represent commercial advantages such as self luminescence and rapid response, thus the organic EL elements are predicted to be widely utilized for flat panel displays. In particular, two-layered or multilayered organic EL elements have been attracting commercial attention, since larger area elements are expected that are capable of emitting light at as low voltage as 10 V or less (see Non-Patent Literature 1, for example). Such multilayered organic EL elements comprise a basic configuration of positive electrode /hole-transporting layer/light-emitting layer/electron-transporting layer/negative electrode, in which the hole-transporting layer or the electron-transporting layer may also perform as the light-emitting layer in the two-layered organic EL element.

Recently, organic EL elements are expected for full-color displays. In the full-color display, pixels showing three primary colors, i.e., blue (B), green (G), and red (R), are necessary to be arranged on a panel. For arranging the pixels, various methods are proposed such as (a) methods of arranging three different organic EL elements emitting blue (B), green (G), and red (R) light, respectively; (b) methods of separating white light (color mixture of blue (B), green (G), and red (R) light emitted from a white-light-emitting organic EL element into the three primary colors using a color filter; and (c) methods of converting blue light from a blue light emitting organic EL element into green (G) light and red (R) light with the use of a color conversion layer utilizing fluorescence emission.

In order to obtain organic EL elements with higher luminous efficiency, an emitting layer is proposed, for example, that is produced from a host material as the main material and a guest material for doping a small amount of dye having a higher fluorescence luminescence (see Non-Patent Literature 2, for example).

However, organic EL elements with sufficient luminous efficiency have not been provided yet in the prior art. Accordingly, we have proposed an organic EL element that comprises 1,3,6,8-tetraphenylpyrene as an emitting material (see Patent Literature 1, for example). In this organic OL element, the emitting luminance is at most about 680 cd/cm² in a condition that a voltage of 10 volts is applied between the negative electrode and the positive electrode; the period for decreasing from initial luminance to half luminance of the initial luminance is 30 hours in a condition that the initial luminance is 150 cd/cm² and the organic EL element is continuously operated under a constant current. As such, our proposed organic EL element is still demanded for higher luminous efficiency and prolonged life time sufficient in display application.

EP-A-1403354 (priority date 28 August 2002; application date 8 August 2003; publication date 31 March 2004) describes 1,3,6,8-tetraphenylpyrene compounds and aryl derivatives, organic EL elements and organic EL displays using the same.

Modrakowski et al. ("Synthesis of Pyrene Containing Building Blocks for Dendrimer Synthesis"; Synthesis 2001, No. 14, 2143-2155) describe a hexyl derivatised 1,3,6,8-tetra(4-biphenyl)pyrene compound for use as a core molecule in the construction of dendrimers.
Non-patent Literature 1: C. W. Tang and S. A. VanSlyke, Applied Physics Letters vol. 51, pp. 913, 1987
Non-Patent Literature 2: C. W. Tang, S. A. VanSlyke, and C. H. Chen, Journal of Applied Physics vol. 65, pp. 3610,1989
Patent Literature 1: Japanese Patent Application Laid-Open (JP-A) No. 2001-118682

### Disclosure of Invention

The object of the present invention is to provide 1,3,6,8-tetrasubstituted pyrene compounds that are suited for a blue light emitting material in organic electroluminescent (EL) elements, organic EL elements that are excellent in luminous efficiency, luminance, and color purity and exhibit long lifetime, and organic EL displays that represent high quality and long lifetime.

The present invention is defined in and by the appended claims. Also described herein is an organic EL element comprising an organic thin layer between a positive electrode and a negative electrode, and the organic thin layer comprises a 1,3,6,8-tetrasubstituted pyrene compound expressed by the formula (1) as the light emitting material,
wherein the organic thin layer comprises a 1,3,6,8-tetrasubstituted pyrene compound, as a light emitting material, expressed by the formula (1): 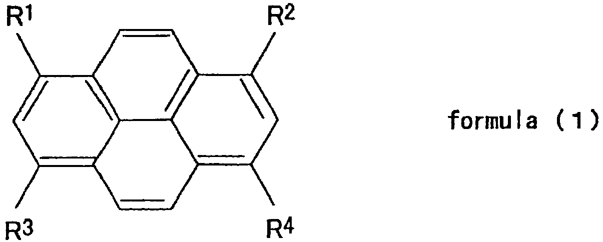 wherein R¹ to R⁴ in the formula (1) may be identical or different each other, and are each a group expressed by the formula (2): 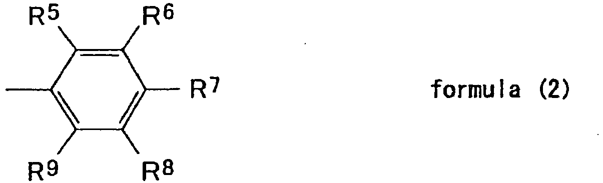 wherein R⁵ to R⁹ in the formula (2) may be identical or different each other, are each a hydrogen atom or a substituted group; and at least one of R⁵ to R⁹ is a substituted or unsubstituted aryl group.

The organic EL element according to the present invention comprises the 1,3,6,8-tetrasubstituted pyrene compounds of the present invention as the emitting material, therefore, the organic EL element according to the present invention may be excellent in luminous efficiency, luminance, and color purity, and may exhibit long lifetime.

A 1,3,6,8-tetrasubstituted pyrene compound may be expressed by the formula (1),
wherein the organic thin layer comprises a 1,3,6,8-tetrasubstituted pyrene compound, as a light emitting material, expressed by the formula (1): 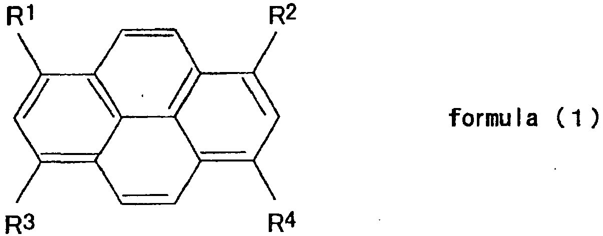 wherein R¹ to R⁴ in the formula (1) may be identical or different each other, and are each a group expressed by the formula (2): 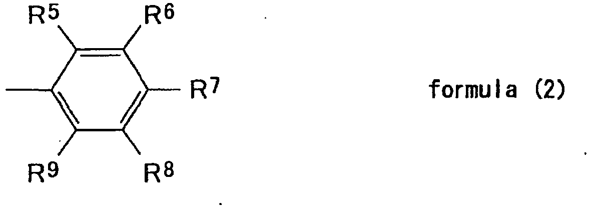 wherein R⁵ to R⁹ in the formula (2) may be identical or different each other, are each a hydrogen atom or a substituted group; and at least one of R⁵ to R⁹ is a substituted or unsubstituted aryl group.

The 1,3,6,8-tetrasubstituted pyrene compound according to the present invention may emit blue light with excellent luminous efficiency, luminance, and color purity, and may exhibit prolonged lifetime.

The organic EL display according to the present invention is formed from the organic EL element according to the present invention. The organic EL display according to the present invention may represent excellent luminous efficiency, luminance, and color purity in blue light, and may exhibit stable performance with time, since it is formed from the organic EL element according to the present invention.

### Brief Description of the Drawings

FIG. 1 is a schematic view that illustrates an exemplary layer configuration of an organic EL element according to the present invention.
FIG. 2 is a schematic view that illustrates an exemplary configuration of an organic EL display in a passive-matrix panel or passive-matrix type.
FIG. 3 is a schematic view that illustrates an exemplary circuit of an organic EL display in a passive-matrix panel or passive-matrix type shown in FIG. 2.
FIG. 4 is a schematic view that illustrates an exemplary configuration of an organic EL display in an active-matrix panel or active-matrix type.
FIG. 5 is a schematic view that illustrates an exemplary circuit of an organic EL display in an active-matrix panel or active-matrix type shown in FIG. 2.
FIG. 6 is an infrared spectrum of resulting synthesized 1,3,6,8-tetra(4-biphenyl)pyrene.
FIG. 7 is an infrared spectrum of resulting synthesized 1,3,6,8-tetra(4-dibenzofuranyl)pyrene.

### Best Mode for Carrying Out the Invention

### < 1,3,6,8-tetrasubstituted pyrene compound >

A 1,3,6,8-tetrasubstituted pyrene compound may be expressed by the formula (1),
wherein the organic thin layer comprises a 1,3,6,8-tetrasubstituted pyrene compound, as a light emitting material, expressed by the formula (1): 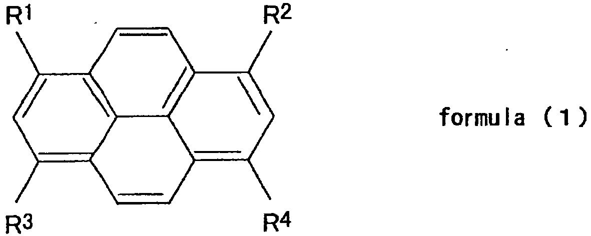 wherein R¹ to R⁴ in the formula (1) may be identical or different each other, and are each a group expressed by the formula (2): 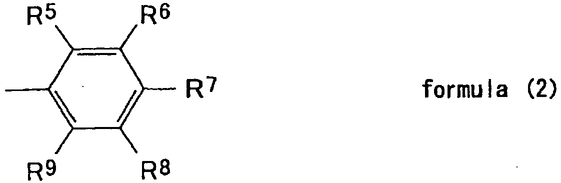 wherein R⁵ to R⁹ in the formula (2) may be identical or different each other, are each a hydrogen atom or a substituted group; and at least one of R⁵ to R⁹ is a substituted or unsubstituted aryl group.

Further, the substituent may be, for example, an alkyl group and an aryl group, and each of these substituents may further be substituted with one or more substituents. The substituents are not specifically limited and may be appropriately selected from known substituents.

The alkyl group described above may be properly selected depending on the application; examples of the alkyl group include, for example, linear, branched-chain or cyclic alkyl groups each having one to ten carbon atoms, specifically, methyl, ethyl, propyl, isopropyl, butyl, isobutyl, t-butyl, pentyl, isopentyl, hexyl, isohexyl, heptyl, isoheptyl, octyl, isooctyl, nonyl, isononyl, decyl, isodecyl, cyclopentyl, cyclobutyl, cyclopentyl, cyclohexyl, cycloheptyl, cyclooctyl, cyclononyl and cyclodecyl.

The aryl group described above may be properly selected depending on the application; for example, preferable are groups having a monocyclic aromatic ring, groups having combined four or less aromatic rings, and groups having fused five or less aromatic rings and containing a total of fifty or less atoms of carbon, oxygen, nitrogen and sulfur atoms.

Examples of the groups having a monocyclic aromatic ring include phenyl, tolyl, xylyl, cumenyl, styryl, mesityl, cinnamyl, phenethyl and benzhydryl. Each of these may be further substituted with one or more substituents.

Examples of the groups having combined four or less aromatic rings include naphthyl, anthryl, phenanthryl, indenyl, azulenyl and benzanthracenyl. Each of these may further be substituted with one or more substituents.

Examples of the groups having fused five or less aromatic rings and containing a total of fifty or less atoms of carbon, oxygen, nitrogen and sulfur atoms include pyrrolyl, furyl, thienyl, pyridyl, quinolyl, isoquinolyl, imidazoyl, pyridinyl, pyrrolopyridinyl, thiazoyl, pyrimidinyl, thiophenyl, indolyl, quinolinyl, purinyl and adenyl. Each of these may be substituted with one or more substituents.

R⁵ to R⁹ in the formula (2) may be connected at least in part to each other directly or indirectly. In such case, R⁵ to R⁹ may be bound to each other with the interposition of at least one atom selected from boron, carbon, nitrogen, oxygen, silicon, phosphorus and sulfur atoms thereby to form a ring such as an aromatic ring, fatty ring, aromatic hetero ring, and hetero ring, and these rings may be further substituted.

When the R¹ to R⁴ in the formula (1), i.e. the groups expressed by the formula (2), are those expressed by the formula (2-1), the 1,3,6,8-tetrasubstituted pyrene compounds are 1,3,6,8-tetra(4-biphenyl)pyrene or the derivatives, 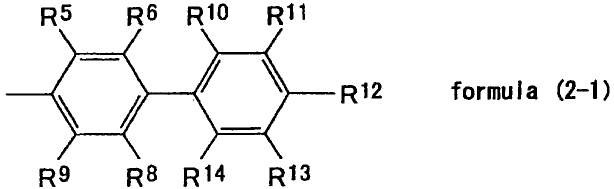 wherein R⁵, R⁶, and R⁸ to R¹⁴ in the formula (2-1) may be identical or different each other, and are each a hydrogen atom or a substituted group. The substituents may be selected from those exemplified above.

One 1,3,6,8-tetrasubstituted pyrene compound of the present invention is 1,3,6,8-tetra(4-biphenyl)pyrene expressed by the formula (1-1).

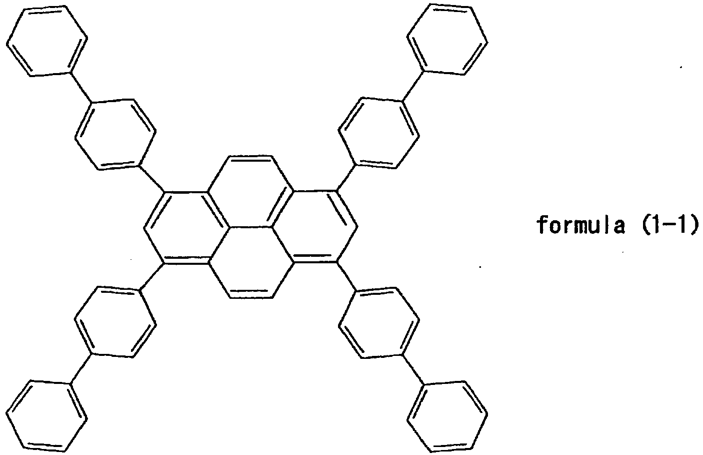

The R¹ to R⁴ in the formula (1), i.e. the groups expressed by the formula (2), may be selected from the groups expressed by the formulas (2-2) to (2-5): 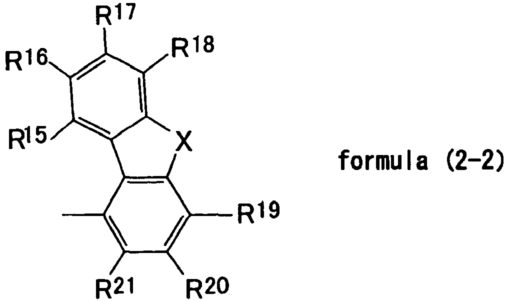 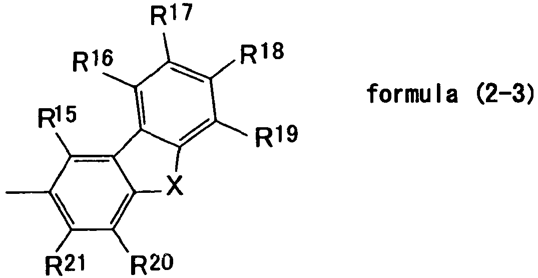 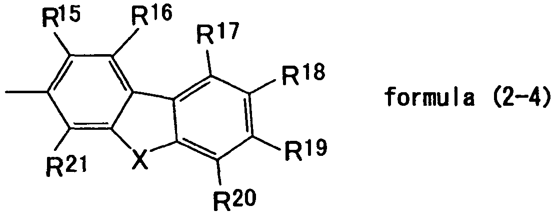 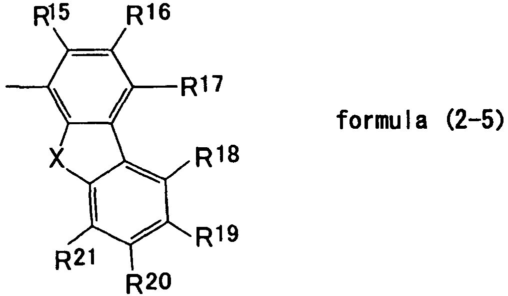 wherein R¹⁵ to R²¹ in the formulas (2-2) to (2-5) may be identical or different each other, are each a hydrogen atom or a substituted group. The substituents may be selected from those exemplified above.

The X in the formulas (2-2) to (2-5) represents a divalent organic group. Examples of the divalent organic group include those expressed by formulas (3) to (6) below: 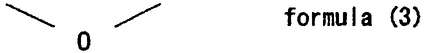 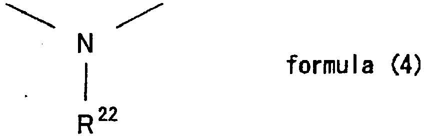 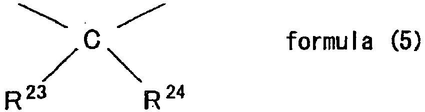 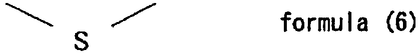 wherein R²² to R²⁴ in the formulas (3) to (6) are each a hydrogen atom or a substituted group. The substituents may be selected from those exemplified above.

Examples of 1,3,6,8-tetrasubstituted pyrene compounds include 1,3,6,8-tetra(4-dibenzofuranyl)pyrene expressed by the formula (1-2) of the present invention, and 1,3,6,8-tetra(4-dibenzothionyl)pyrene expressed by the formula (1-3).

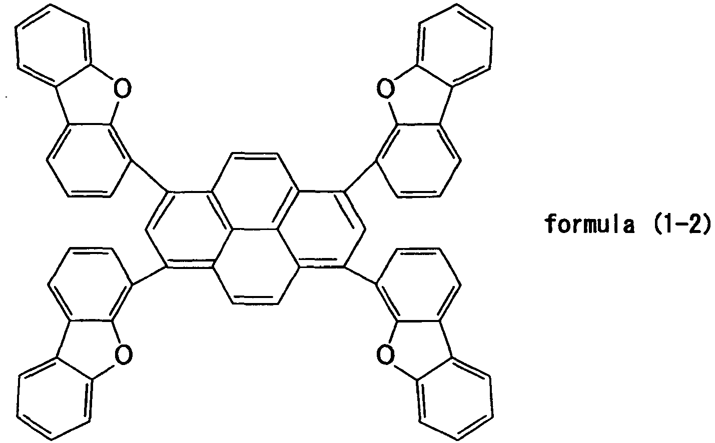

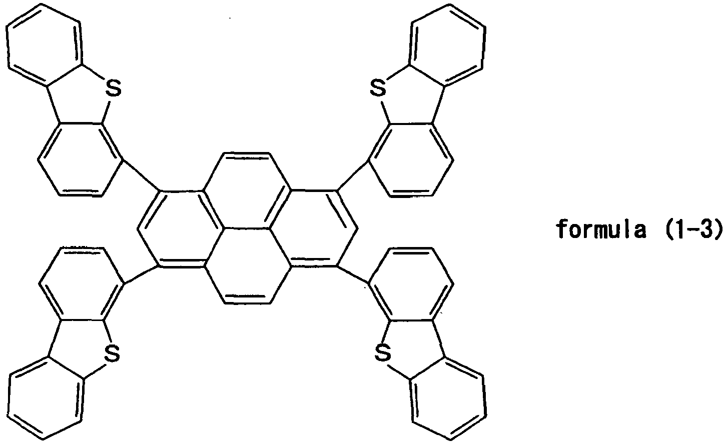

The process for producing the 1,3,6,8-tetrasubstituted pyrene compounds according to the present invention may be properly selected depending on the application; preferable example of the process is as follows.

Initially, one equivalent of pyrene and four equivalents of halogen are reacted to synthesize 1,3,6,8-tetrahalogenated pyrene. The tetrahalogenation of pyrene inherently tends to yield at 1, 3, 6, and 8 sites. Preferably, the halogenation is carried out substantially according to typical halogenation process of usual aromatic hydrocarbons as illustrated in "Annalen der Chemie vol. 531, page 81" such that pure halogen is added to pyrene dissolved in a solvent. Preferable halogens are chlorine, bromine, and iodine so as to advantageously carry out the subsequent reaction; and chlorine or bromine is more preferable from the viewpoint of easy halogenation.

Then, 1,3,6,8-tetrahalogenated peropyrene and arylboronic acid, which corresponds to the intended compound, are heated under the presence of a catalyst and a basic substance to synthesize the inventive 1,3,6,8-tetrahalogenated peropyrene by reaction of so-called Suzuki coupling. The catalyst may be palladium compounds such as tetrakis(triphenylphosphine)palladium (0). The basic substance may be selected from sodium carbonate, potassium carbonate, sodium hydroxide, and sodium alkoxide such as sodium tert-butoxide, for example.

Specifically, in order to synthesize 1,3,6,8-tetra(4-biphenylyl)pyrene in accordance with the typical process explained above, initially, pyrene and bromine is reacted to produce 1,3,6,8-tetrabromopyrene. Then, 1,3,6,8-tetrabromopyrene is subjected to a reaction under so-called Suzuki coupling to synthesize 1,3,6,8-tetra(4-biphenylyl)pyrene. Namely, 4.4 equivalents of 4-biphenylboronic acid expressed by the following formula, 10 equivalents of sodium carbonate as a solution of 2 mole/liter-water, and 0.12 equivalent of tetrakis(triphenylphosphine)palladium (0) are added to one equivalent of 1,3,6,8-tetrabromopyrene, then the mixture is refluxed for about 3 hours using benzene as a solvent under heating to react these compounds. Following the reaction, the resulting product is cooled and rinsed several times by water; and the benzene is distilled away. The remaining oily substance is rinsed by methanol, then is recrystallized using a mixed solvent of tetrahydrofuran and methanol thereby to produce a raw reaction product. The raw reaction product is purified by means of vacuum sublimation to obtain the intended 1,3,6,8-tetra(4-biphenylyl)pyrene.

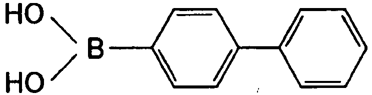

Further, in order to synthesize 1,3,6,8-tetra(4-dibenzofuranyl)pyrene, initially, pyrene and bromine is reacted to produce 1,3,6,8-tetrabromopyrene. Then, 1,3,6,8-tetrabromopyrene is subjected to a reaction under so-called Suzuki coupling to synthesize 1,3,6,8-tetra(4-biphenylyl)pyrene. Namely, 4.4 equivalents of dibenzofuranboronic acid expressed by the following formula, 10 equivalents of sodium carbonate as a solution of 2 mole/liter-water, and 0.12 equivalent of tetrakis(triphenylphosphine)palladium (0) are added to one equivalent of 1,3,6,8-tetrabromopyrene, then the mixture is refluxed for about 3 hours using benzene as a solvent under heating to react these compounds. Following the reaction, the resulting product is cooled and rinsed several times by water; and the benzene is distilled away. The remaining oily substance is rinsed by methanol, then is recrystallized using a mixed solvent of tetrahydrofuran and methanol thereby to produce a raw reaction product. The raw reaction product is purified by means of vacuum sublimation to obtain the intended 1,3,6,8-tetra(4-dibenzofuranyl)pyrene.

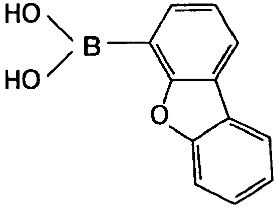

The 1,3,6,8-tetrasubstituted pyrene compounds according to the present invention may be advantageously utilized in various commercial fields, typically as light emitting materials in organic EL elements. The 1,3,6,8-tetrasubstituted pyrene compounds according to the present invention emit blue light when employed as emitting materials in organic EL elements.

### <Organic EL Element>

The organic EL elements according to the present invention comprise a positive electrode, a negative electrode, and an organic thin layer arranged between the positive electrode and the negative electrode, in which the organic thin layer comprises the 1,3,6,8-tetrasubstituted pyrene compounds according to the present invention, namely, the 1,3,6,8-tetrasubstituted pyrene compounds expressed by the formulae (1-1) and (1-2) as a light emitting material.

The 1,3,6,8-tetrasubstituted pyrene compound incorporated as a light emitting material in the organic thin layer may be contained in a light emitting layer, alternatively in a light-emitting electron-transporting layer which is a light emitting layer as well as a electron transporting layer or in a light-emitting hole-transporting layer which is a light emitting layer as well as a hole transporting layer, of the organic thin layer. When the 1,3,6,8-tetrasubstituted pyrene compound is contained in the light-emitting layer, the light-emitting layer may comprise the 1,3,6,8-tetrasubstituted pyrene alone or may further comprise other material in addition to the 1,3,6,8-tetrasubstituted pyrene compound.

Preferably, the light-emitting layer, light-emitting electron-transporting layer, or light-emitting hole-transporting layer in the organic thin layer contains the inventive 1,3,6,8-tetrasubstituted pyrene compound as a guest material and further contains, in addition to the guest material, a host material capable of emitting light with a wavelength near to the absorption wavelength of the guest material. Preferably, the host material is contained in the light-emitting layer; or the host material may be contained in the hole-transporting layer, the electron-transporting layer, or the like.

In the condition that the guest material and the host material are used in combination, the host material is initially excited when organic electroluminescence is induced. The excitation energy efficiently moves from the host material to the guest material, because the emission wavelength of the host material overlaps the absorption wavelength (330 to 600 nm) of the guest material (1,3,6,8-tetrasubstituted pyrene compound). Thus, the host material returns to a ground state without light emission, and the guest material in an excited state alone emits the excitation energy as blue light. This configuration may therefore provide excellent emission efficiency, emission luminance, and color purity of blue light.

In general, when luminescent molecules are contained alone or at high concentration in a thin film, the luminescent molecules tend to interact each other to cause a drop of emission efficiency, which is a phenomenon called as "concentration quenching". On the contrary, when the guest material and the host material are combined, the 1,3,6,8-tetrasubstituted pyrene compound as the guest compound is dispersed in a relatively low concentration with the host compound, and the "concentration quenching" may be effectively prevented, resulting in advantageously high emission efficiency. The combination of the guest material and the host material is typically advantageous for the light-emitting layer, since the host material generally provide proper film-forming property, thus the light-emitting layer may be formed successfully while maintaining the excellent emission properties.

The host material may be properly selected depending on the application; preferably, the host material has an emission wavelength in the vicinity of the optical absorption wavelength of the guest material. Preferable examples of the host material include aromatic amine derivatives expressed by following formula (7); carbazole derivatives expressed by following formula (8); hydroxyquinoline oxyaryl complexs expressed by following formula (11); 1,3,6,8-tetraphenylpyrene compounds expressed by following formula (13); 4,4'-bis(2,2'-diphenylvinyl)-1,1'-biphenyl (DPVBi) expressed by following formula (15) having a main emission wavelength of 470 nm; p-sesquiphenyl expressed by following formula (16) having a main emission wavelength of 400 nm; and 9,9'-bianthryl expressed by following formula (17) having a main emission wavelength of 460 nm.

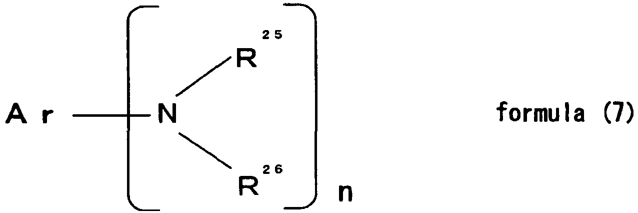

In formula (7), "n" is an integer of 2 or 3; Ar represents a divalent or trivalent aromatic or heteroaromatic group; and R²⁵ and R²⁶ may be identical or different from each other and each represents a monovalent aromatic or heteroaromatic group. The monovalent aromatic or heteroaromatic group may be properly selected depending on the application.

Among the aromatic amine derivatives expressed by formula (7), N,N'-dinaphthyl-N,N'-diphenyl-[1,1'-biphenyl]-4,4'-diamine (NPD) expressed by following formula (8) having a main emission wavelength of 430 nm and derivatives thereof are preferable, 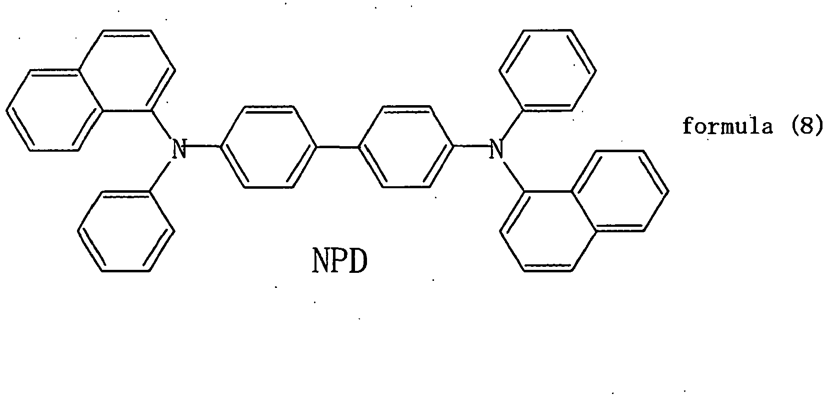 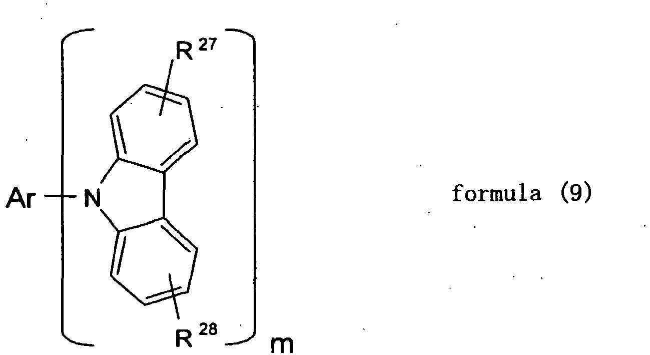 wherein Ar in formula (9) represents a divalent or trivalent group containing an aromatic ring or a heteroaromatic group, 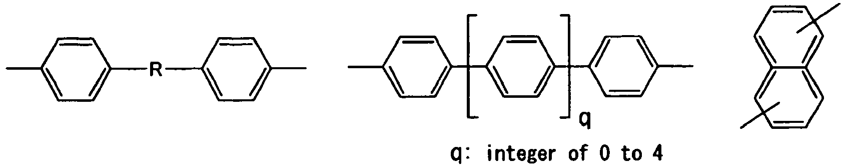 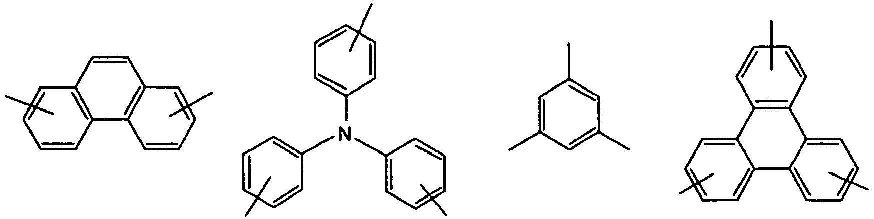 wherein these groups may be further substituted by a unconjugated group; the R in the above group represents a connecting group, preferable examples thereof include the followings.

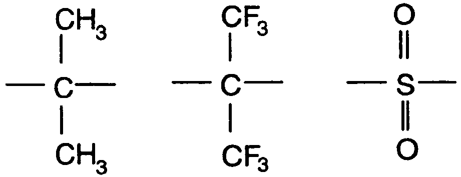

In the formula (9), R²⁷ and R²⁸ may be independently one of hydrogen atom, halogen atoms, alkyl groups, aralkyl groups, alkenyl groups, aryl groups, cyano groups, amino groups, acyl groups, alkoxycarbonyl groups, carboxyl group, alkoxy groups, alkylsulfonyl groups, hydroxy group, amido groups, aryloxy group, aromatic cyclic hydrocarbon groups, heteroaromatic groups, and substituted groups thereof; and "m" is an integer of 2 or 3.

Among the aromatic amine derivatives expressed by formula (9), the compound of which Ar is an aromatic group comprising two benzene rings bound each other with interposition of a single bond, R²⁷ and R²⁸ are each a hydrogen atom, and "m" is 2; namely, 4,4'-bis(9-carbazolyl)-biphenyl (CBP) expressed by following formula (10) having a main emission wavelength of 380 nm, and a derivative thereof are preferable for excellent emission efficiency, emission luminance, and color purity of blue light, 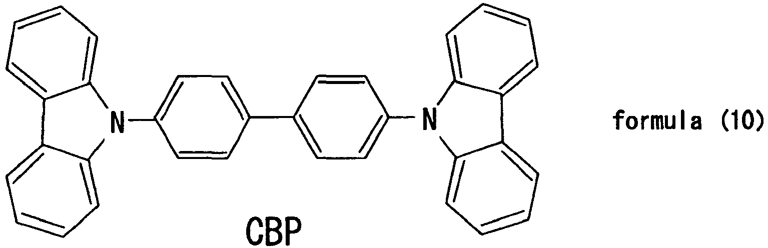 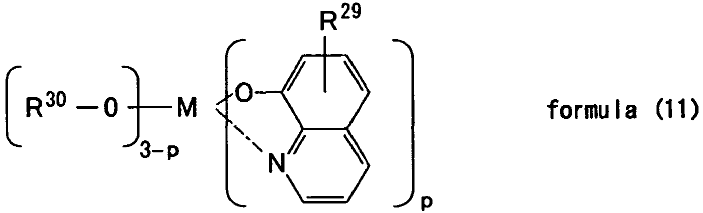 wherein M represents a trivalent metal atom; R²⁹ represents a hydrogen atom or an alkyl group; R³⁰ represents a hydrogen atom or an aryl group; and "p" is an integer of 1 or 2.

Among the hydroxyquinoline oxyaryl complexs expressed by formula (11), aluminum hydroxyquinoline oxybiphenyl complex (BAlq) expressed by formula (12) is preferable.

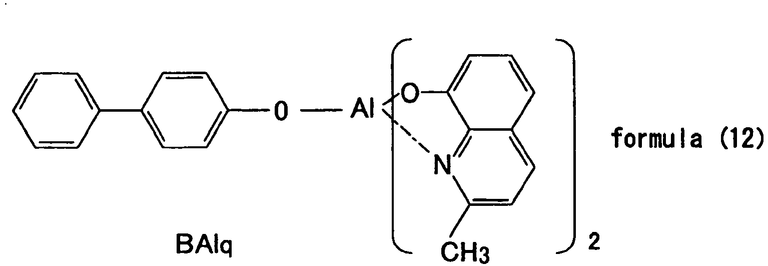

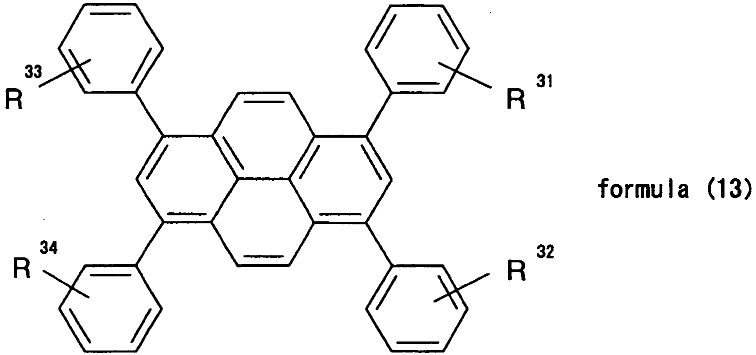

In the formula (13), R³¹ to R³⁴ may be identical or different each other and are each a hydrogen atom or substituent. The substituent are preferably an alkyl group, cycloalkyl group, or aryl group; and these may be further substituted.

Among the 1,3,6,8-tetraphenylpyrenes expressed by formula (13), the compound in which R³¹ to R³⁴ are hydrogen atoms, namely, 1,3,6,8-tetraphenylpyrene expressed by following formula (14) having a main emission wavelength of 440 nm is preferable from the viewpoint of excellent emission efficiency, emission luminance, and color purity of blue light.

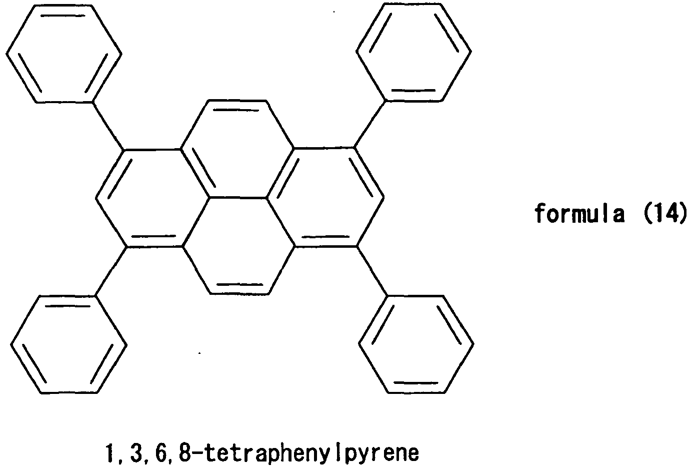

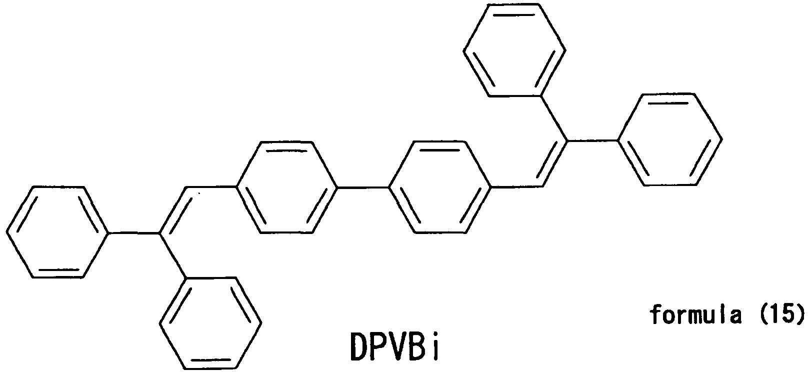

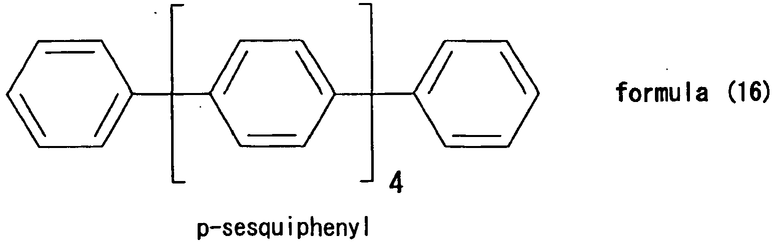

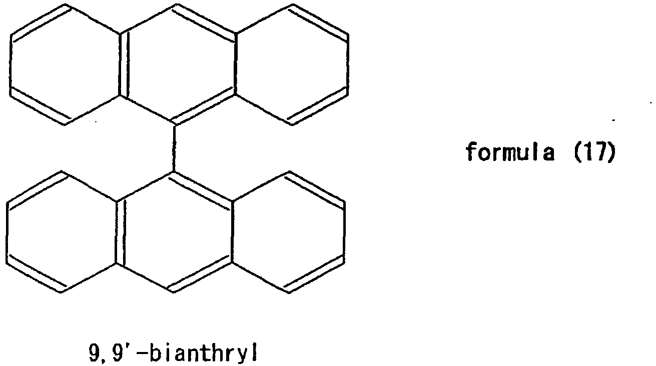

The content of the 1,3,6,8-tetrasubstituted pyrene compound is preferably 0.1 to 50 percent by mass, more preferably 0.5 to 20 percent by mass in the layer that contains 1,3,6 8-tetrasubstituted pyrene compound expressed by the formulae (1-1) and (1-2). When the content is less than 0.1 percent by mass, the emission efficiency, emission luminance, color purity etc. may be insufficient; and when the content is above 50 percent by mass, the color purity may be lower. In contrast, the content within the range indicated above is advantageous for excellent emission efficiency, emission luminance, and color purity.

The light emitting layer in the organic EL element according to the present invention may receive holes from a positive electrode, hole injecting layer, or hole transporting layer when an electric field is applied, and also may receive electrons from a negative electrode, electron injecting layer, or electron transporting layer; thus, the light emitting layer may provide a field of recombination between the holes and the electrons and may enable the 1,3,6,8-tetrasubstituted pyrene compound, i.e. emitting material and luminescent molecules, to emit blue light by the action of recombination energy generated by the recombination. The light emitting layer may further comprise other light emitting materials in addition to 1,3,6,8-tetrasubstituted pyrene compound within a range not deteriorating the blue light emission.

The light emitting layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

Among them, vapor deposition process is typically proper, since organic solvents are not necessary and thus is free from the waste products of the solvents, the cost is lower, and the production efficiency is higher. By the way, wet forming process is also preferable when the light emitting layer is of single layer configuration such as a hole-transporting light-emitting electron-transporting layer.

More specifically, the vapor deposition process may be properly selected depending on the application; preferable are, but not limited to, vacuum vapor deposition, resistance heating vapor deposition, chemical vapor deposition, and physical vapor deposition. Specific examples of the chemical vapor deposition (CVD) include plasma CVD, laser CVD, thermal CVD, and gas source CVD. The light emitting layer may be formed by means of the vapor deposition through subjecting the 1,3,6,8-tetrasubstituted pyrene compound to vacuum vapor deposition, for example. When the light emitting layer comprises the host material in addition to the 1,3,6,8-tetrasubstituted pyrene compound, the 1,3,6,8-tetrasubstituted pyrene compound and the host material are subjected simultaneous vacuum vapor deposition. The former process may typically produce the layer relatively easily, since co-vapor deposition is not required.

The wet forming process may be properly carried out according to the intended layer. Examples of the procedure include ink jet process, spin coating process, kneader coating process, bar coating process, blade coating process, casting process, dipping process, and curtain coating process.

According to the wet forming process, a solution may be utilized that comprises raw materials for the light emitting layer as well as resin components dissolved or dispersed in the solution. Examples of the resin components include polyvinylcarbazoles, polycarbonates, polyvinyl chlorides, polystyrenes, polymethylmethacrylates, polyesters, polysulfones, polyphenylene oxides, polybutadienes, hydrocarbon resins, ketone resins, phenoxy resins, polyamides, ethyl celluloses, vinyl acetates, ABS resins, polyurethanes, melamine resins, unsaturated polyester resins, alkyd resins, epoxy resins, and silicone resins.

The light emitting layer may be appropriately prepared by the wet forming process, for example, by means of a solution of coating composition that contains the 1,3,6,8-tetrasubstituted pyrene compound and the optional resin material dissolved in a solvent, by applying and drying the coating composition. When the light emitting layer comprises the host material in addition to the 1,3,6,8-tetrasubstituted pyrene compound, the light emitting layer may be prepared from a solution of coating composition that comprises the 1,3,6,8-tetrasubstituted pyrene compound, the host material, and the optional resin material in a solvent, by applying and drying the coating composition. The thickness of the light emitting layer is preferably 1 to 50 nm, and more preferably is 3 to 20 nm.

The thickness of the light emitting layer within the indicated range may provide sufficient emission efficiency, emission luminance, and color purity of blue light emitted by the organic EL element. These advantages are more significant when the thickness is within the more preferable range.

The organic EL element according to the present invention comprises a positive electrode, a negative electrode, and an organic thin layer containing a light emitting layer, and is arranged between a positive electrode and a negative electrode and may further comprise other layers such as a protective layer.

The organic thin layer comprises at least a light emitting layer and may further comprise other layers such as a hole injecting layer, hole transporting layer, hole blocking layer, electron transporting layer, and electron injecting layer.

### - Positive Electrode -

The positive electrode may be properly selected depending on the application; preferably, the positive electrode is one capable of supplying holes or carriers to the organic thin layer. More specifically, the positive electrode is preferably capable of supplying carriers to the light emitting layer when the organic thin layer comprises the light emitting layer alone, to the hole transporting layer when the organic thin layer further comprises the hole transporting layer, and to the hole injecting layer when the organic thin layer further comprises the hole injecting layer.

The material for the positive electrode may be properly selected depending on the application; examples thereof include metals, alloys, metal oxides, electroconductive compounds, and mixtures of these materials. Among them, such materials are preferable that have a work function of 4 eV or more.

Specific examples of the material for the positive electrode are electroconductive metal oxides such as tin oxide, zinc oxide, indium oxide, and indium tin oxide (ITO); metals such as gold, silver, chromium, and nickel; mixtures or laminates of these metals and electroconductive metal oxides; inorganic electroconductive materials such as copper iodide and copper sulfide; organic electroconductive materials such as polyanilines, polythiophenes, and polypyrroles; and laminates of these materials with ITO. These may be used alone or in combination. Among them, electroconductive metal oxides are preferable, and ITO is specifically preferable for superior productivity, high conductivity, and transparency.

The thickness of the positive electrode may be properly selected depending on the application and the material; preferably, the thickness is 1 to 5000 nm, and more preferably is 20 to 200 nm from the viewpoint of electric resistivity and optical absorption.

The positive electrode is typically arranged on a substrate made of, for example, glasses such as soda lime glass and non-alkali glass, or transparent resins.

The glass for the substrate is preferably non-alkali glass or soda lime glass having a barrier coating such as silica coating for reducing migration ions dissolved from the glass.

The thickness of the substrate is not specifically limited, as long as the substrate maintains a certain mechanical strength. When a glass is used as the substrate, the thickness is typically 0.2 mm or more and preferably 0.7 mm or more.

The positive electrode may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

The drive voltage may be decreased and/or the emission efficiency may be increased by subjecting the positive electrode to rinsing or other treatments. Suitable examples of the other treatments include UV-ozone treatment and plasma treatment when the positive electrode is formed from ITO.

### - Negative Electrode -

The negative electrode may be properly selected depending on the application; preferably, the negative electrode is capable of supplying electrons. More specifically, the negative electrode is preferably capable of supplying electrons to the light emitting layer when the organic thin layer contains solely the light emitting layer, to the electron transporting layer when the organic thin layer further contains the electron transporting layer, and to an electron injecting layer when the organic thin layer contains the electron injecting layer between the organic thin layer and the negative electrode.

The material for the negative electrode may be appropriately selected typically depending on such factors as adhesion properties with layers or molecules adjacent to the negative electrode, e.g. the electron transporting layer and/or the light emitting layer, and also ionization potential, and stability. Examples of the material include metals, alloys, metal oxides, electroconductive compounds, and mixtures thereof.

Specific examples of the material for the negative electrode include alkali metals such as Li, Na, K and Cs; alkaline earth metals such as Mg and Ca; gold, silver, lead, aluminum, sodium-potassium alloys or mixed metals thereof, lithium-aluminum alloys or mixed metals thereof, magnesium-silver alloys or mixed metals thereof; rare earth metals such as indium and ytterbium; and alloys of these metals.

These materials may be used alone or in combination. Among them, materials having a work function of 4 eV or less are preferable, and more preferable are aluminum, lithium-aluminum alloy or mixed metals thereof, magnesium-silver alloy, or mixed metals thereof.

The thickness of the negative electrode may be properly selected depending on the material of the negative electrode; preferably, the thickness is 1 to 10000 nm, and more preferably is 20 to 200 nm.

The negative electrode may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

When two or more different materials are used for the negative electrode, the two or more different materials may be subjected to vapor deposition simultaneously to form an alloy electrode, alternatively a preformed alloy may be subjected to vapor deposition to form an alloy electrode, for example.

Preferably, the resistance of the positive electrode and the negative electrode is as low as possible, and is several hundred ohms per square or less.

### - Hole Injecting Layer -

The hole injecting layer may be properly selected depending on the application; preferably, the hole injecting layer is capable of injecting holes from the positive electrode when an electric field is applied.

The material for the hole injecting layer may be properly selected depending on the application; and suitable examples of the material include the starburst amine (4,4',4"-tris[3-methylphenyl(phenyl)amino]triphenylamine: m-MTDATA) expressed by the following formula, copper phthalocyanine, and polyanilines.

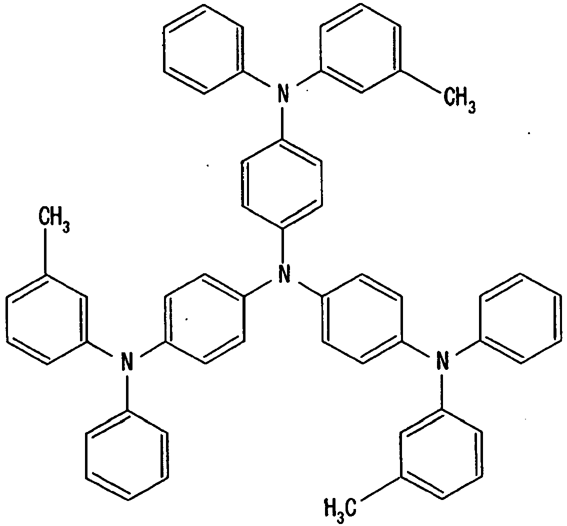

The thickness of the hole injecting layer may be properly selected depending on the application; preferably, the thickness is about 1 to 100 nm, and more preferably is 5 to 50 nm.

The hole injecting layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

### - Hole Transporting Layer -

The hole transporting layer may be properly selected depending on the application; preferably, the hole transporting layer is capable of transporting holes from the positive electrode when an electric field is applied.

The material for the hole transporting layer may be properly selected depending on the application; examples of the material include aromatic amine compounds, carbazole, imidazole, triazole, oxazole, oxadiazole, polyarylalkanes, pyrazoline, pyrazolone, phenylenediamine, arylamines, amino-substituted chalcones, styrylanthracene, fluorenone, hydrazone, stilbene, silazane, styrylamine, aromatic dimethylidene compounds, porphyrin compounds, polysilane compounds, poly(N-vinylcarbazole)s, aniline copolymers, thiophene oligomers and polymers, polythiophenes and other electroconductive high-molecular oligomers and polymers and carbon films. By the way, when the material of the hole transporting layer and the material of the light emitting material are blended to form a layer, the layer may be a hole-transporting light-emitting layer.

These may be used alone or in combination. Among them, aromatic amine compounds are preferable, more preferably are TPD (N,N'-diphenyl-N,N'-bis(3-methylphenyl)-[1,1'-biphenyl]-4,4'-diamine) and NPD (N,N'-dinaphthyl-N,N'-diphenyl-[1,1'-biphenyl]-4,4'-diamine) expressed by the following formulas.

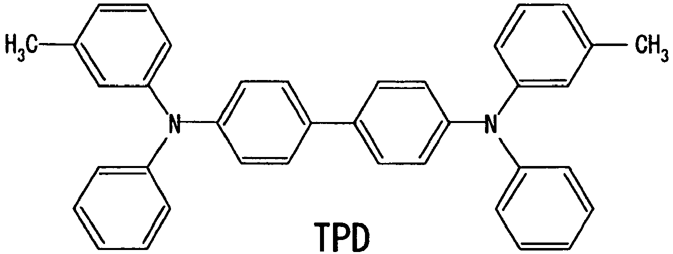

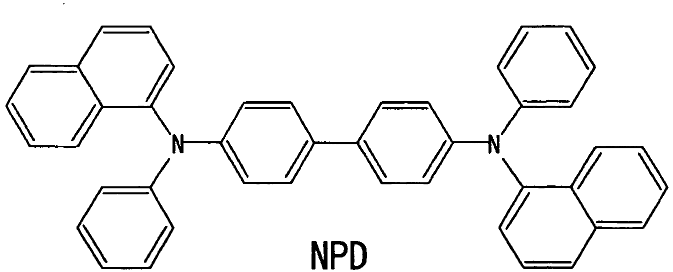

The thickness of the hole transporting layer may be properly selected depending on the application; the thickness is preferably 1 to 500 nm, and more preferably is 10 to 100 nm.

The hole transporting layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

### - Hole Blocking Layer -

The hole blocking layer may be properly selected depending on the application; preferably, the hole blocking layer is capable of blocking holes injected from the positive electrode. The material for the hole blocking layer may be properly selected depending on the application.

When the organic EL element comprises the hole blocking layer, holes transported from the positive electrode are blocked by the hole blocking layer, and electrons transported from the negative electrode pass through the hole blocking layer and arrive at the light emitting layer. Thus, since the holes efficiently recombine with the electrons in the light emitting layer, the recombination between the holes and the electrons in the other areas of the organic thin layer than the light emitting layer is efficiently prevented, and the target 1,3,6,8-tetrasubstituted pyrene compound, as a light emitting material, may emit light with excellent color purity.

Preferably, the hole blocking layer is arranged between the light emitting layer and the electron transporting layer.

The thickness of the hole blocking layer may be properly selected depending on the application; the thickness is preferably about 1 to 500 nm, and more preferably is 10 to 50 nm. The hole blocking layer may be of single layer or multilayered configuration.

The hole blocking layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

### - Electron Transporting Layer -

The electron transporting layer may be properly selected depending on the application; preferably, the electron transporting layer is capable of transporting electrons from the negative electrode and/or capable of blocking holes injected from the positive electrode.

The material for the electron transporting layer may be properly selected depending on the application; examples of the material include quinoline derivatives such as the aluminum quinoline complex (Alq), oxadiazole derivatives, triazole derivatives, phenanthroline derivatives, perylene derivatives, pyridine derivatives, pyrimidine derivatives, quinoxaline derivatives, diphenylquinone derivatives, and nitro-substituted fluorene derivatives. By the way, when the material of the electron transporting layer and the material of the light emitting material are blended to form a layer, the layer may be an electron-transporting light-emitting layer, and when the material of the hole transporting material is blended further, the layer may be an electron-transporting hole-transporting light-emitting layer; for the purpose of forming such a layer, polymers such as polyvinylcarbazoles or polycarbonates may be employed appropriately.

The thickness of the electron transporting layer may be properly selected depending on the application; the thickness is preferably about 1 to 500 nm, and more preferably is 10 to 50 nm. The electron transporting may be of single layer or multilayered configuration.

The electron transporting material for the electron transporting layer arranged adjacent to the light emitting layer is preferably one having an optical absorption range of wavelength shorter than that of the 1,3,6,8-tetrasubstituted pyrene compound, from the viewpoint that light emitting region in the organic EL element is defined to the light emitting layer and extra light emission is prevented from the electron. Examples of the electron transporting material having an optical absorption range of wavelength shorter than that of the 1,3,6,8-tetrasubstituted pyrene compound include phenanthroline derivatives, oxadiazole derivatives, triazole derivatives, 2,9-dimethyl-4,7-diphenyl-1,10-phenanthroline (BCP), 2-(4-tert-butylphenyl)-5-(4-biphenylyl)-1,3,4-oxadiazole, 3-phenyl-4-(1-naphthyl)-5-phenyl-1,2,4-triazole, and 3-(4-tert-butylphenyl)-4-phenyl-5-(4'-biphenylyl)-1,2,4-triazole.

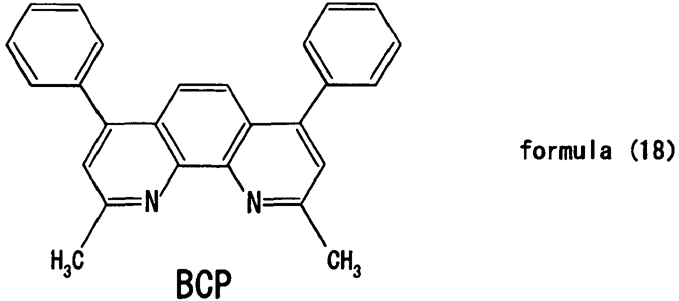

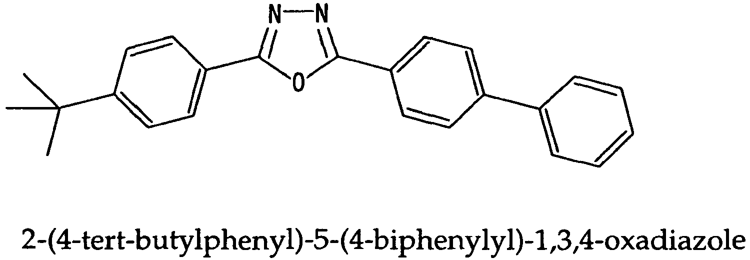

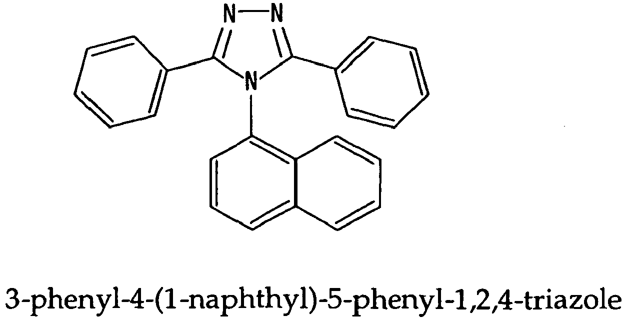

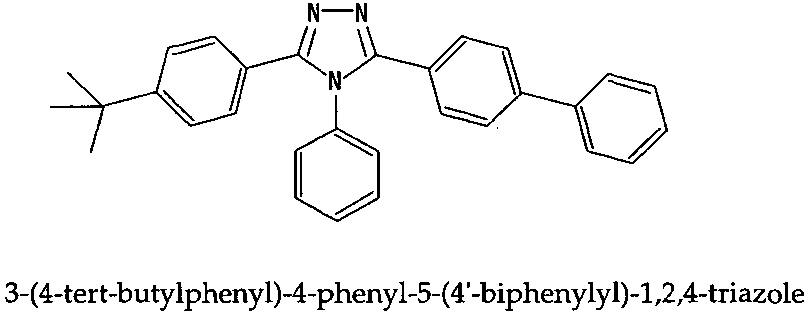

The electron transporting layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

### - Electron Injecting Layer -

The electron injecting layer may be properly selected depending on the application; preferably, the electron injecting layer is capable of injecting electrons from the negative electrode to the other material and capable of sending the electrons to the electron transporting layer.

The material of the electron injecting layer may be alkali metal fluorides such as lithium fluoride and alkaline earth metal fluorides such as strontium fluoride. The thickness of the electron injecting layer may be properly selected depending on the application; the thickness is preferably 0.1 to 10 nm, more preferably is 0.5 to 2 nm from the view point of easy electron injection into the organic thin layer.

The electron injecting layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

### - Other Layers -

The organic EL element according to the present invention may further comprise other layers depending on the application; an example of the other layers is a protective layer.

The protective layer may be properly selected depending on the application; preferably, the protective layer is capable of preventing molecules or substance, which deteriorates the organic EL element such as moisture or oxygen, from entering into the organic EL element.

Examples of the material for the protective layer include metals such as In, Sn, Pb, Au, Cu, Ag, Al, Ti, and Ni; metal oxides such as MgO, SiO, SiO₂, Al₂O₃, GeO, NiO, CaO, BaO, Fe₂O₃, Y₂O₃, and TiO₂; nitrides such as SiN and SiNxOy; metal fluorides such as MgF₂, LiF, AlF₃, and CaF₂; polyethylenes, polypropylenes, polymethylmethacrylates, polyimides, polyureas, polytetrafluoroethylenes, polychlorotrifluoroethylenes, polydichlorodifluoroethylenes, copolymers of chlorotrifluoroethylene and dichlorodifluoroethylene, copolymers prepared by copolymerizing a monomer mixture of tetrafluoroethylene and at least a comonomer, fluorine-containing copolymers having a cyclic structure in a backbone chain thereof, water absorbing substances having a water absorbing capacity of 1% or more, and moisture-proof substances having a water absorbing capacity of 0.1% or less.

The protective layer may be formed, for example, by various processes such as vapor deposition process, wet forming process, electron beam process, sputtering process, reactive sputtering process, molecular beam epitaxy (MBE) process, ionized cluster beam process, ion plating process, plasma polymerization process or high-frequency excitation ion plating process, molecular stacking process, Langmuir-Blodgett (LB) process, printing process, transfer printing process, and chemical reaction process such as sol-gel process by coating ITO dispersion.

The configuration of the organic EL element according to the present invention may be properly selected depending on the application. Suitable examples of the layer configuration are the following layer configurations (1) to (13); that is, (1) positive electrode /hole-injecting layer/hole-transporting layer/light-emitting layer/electron-transporting layer/electron-injecting layer/negative electrode, (2) positive electrode/hole-injecting layer/hole-transporting layer/light-emitting layer/electron-transporting layer/negative electrode, (3) positive electrode/hole-transporting layer/light-emitting layer/electron-transporting layer/electron-injecting layer/negative electrode, (4) positive electrode/hole-transporting layer/light-emitting layer/electron-transporting layer/negative electrode, (5) positive electrode /hole-injecting layer /hole-transporting layer/light-emitting electron-transporting layer/electron-injecting layer/negative electrode, (6) positive electrode/hole-injecting layer /hole-transporting layer/light-emitting electron-transporting layer/negative electrode, (7) positive electrode/hole-transporting layer/light-emitting electron-transporting layer/electron-injecting layer/negative electrode, (8) positive electrode/hole-transporting layer/ light-emitting electron-transporting layer/negative electrode, (9) positive electrode/hole-injecting layer /hole-transporting light-emitting layer/electron-transporting layer/electron-injecting layer/negative electrode, (10) positive electrode/hole-injecting layer /hole-transporting light-emitting layer/electron-transporting layer/negative electrode, (11) positive electrode/hole-transporting light-emitting layer/electron-transporting layer/electron-injecting layer/negative electrode, (12) positive electrode/hole-transporting light-emitting layer/electron-transporting layer/negative electrode, and (13) positive electrode/hole-transporting light-emitting electron-transporting layer/negative electrode.

When the organic EL element further comprises the hole-blocking layer, the hole-blocking layer is preferably arranged between the light-emitting layer and the electron-transporting layer in the layer configurations (1) to (13).

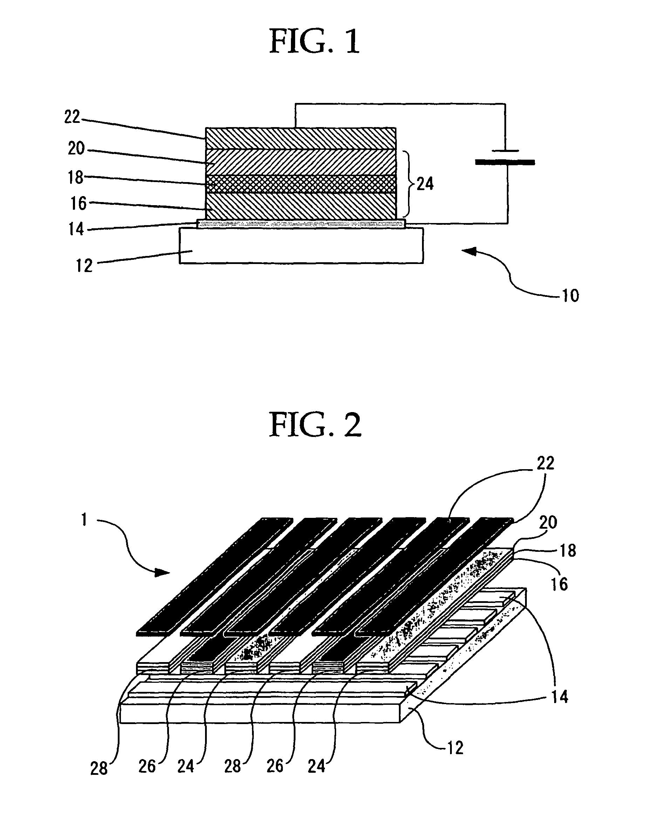

Among these layer configurations, an aspect of the layer configuration (4) positive electrode/hole-transporting layer/light-emitting layer/electron-transporting layer/negative electrode is illustrated in FIG. 1. The organic EL element 10 has a layer configuration comprising glass substrate 12, positive electrode 14 of ITO electrode for example, hole-transporting layer 16, light-emitting layer 18, electron-transporting layer 20, and negative electrode 22 of Al-Li electrode for example arranged in this order. The positive electrode 14 and the negative electrode 22 are connected to each other through a power source. The hole-transporting layer 16, the light-emitting layer 18, and the electron-transporting layer 20 constitute organic thin layer 24 for emitting blue light.

Preferably, the peak emission wavelength of the organic EL element according to the present invention is 400 to 480 nm.

With respect to emission efficiency, the organic EL element according to the present invention is preferably capable of emitting blue light at voltages of 10 V or less, more preferably at voltages of 7 V or less, and specifically preferably at voltages of 5 V or less from the view point of practical applications.

The emission luminance of the organic EL element according to the present invention is preferably 100 cd/m² or more, more preferably is 500 cd/m² or more, and still more preferably is 1000 cd/m² or more at applying a voltage of 10 Volts from the view point of practical applications.

The organic EL elements according to the present invention may be appropriately utilized for various apparatuses or devices such as computers, on-vehicle displays, outdoor displays, household appliances, commercial equipment, household electric equipment, traffic displays, clock displays, calendar displays, luminescent screens, and audio equipment; in addition, may be preferably utilized for the organic EL displays according to the present invention.

### <Organic EL Display>

The organic EL (electroluminescent) display according to the present invention may be properly constructed without particular limitations, provided that the organic EL display comprises the organic EL element according to the present invention. The organic EL display may be of single blue color, plural colors, or full color.

With respect to methods for providing the full-color organic EL display, the representative methods are, as illustrated in "Monthly Display, September 2000 issue, pages 33-37", three-color light emitting methods in which organic EL elements each emitting light corresponding to the three primary colors, red (R), green (G), or blue (B) light, are disposed on a substrate; white color methods in which white light from a white light emitting organic EL element is separated into three primary colors through a color filter; and color conversion methods in which blue light from a blue light emitting organic EL element is converted into red (R) and green (G) colors through a fluorescent dye layer. Since the organic EL element according to the present invention is utilized for emitting blue light, the three-color light emitting method or the color conversion method is preferably employed, and the three-color light emitting method is specifically preferably employed in the present invention.

Providing a full-color organic EL display by the three-color light emitting method requires an organic EL element for emitting green light and an organic EL element for emitting red light, in addition to the organic EL element according to the present invention for emitting blue light.

The organic EL element for emitting red light may be properly selected depending on the application; and is preferably one having a layer configuration of ITO (positive electrode)/NPD/DCJTB expressed by the formula below 1% Al quinoline complex (Alq)/Alq/Al-Li (negative electrode).

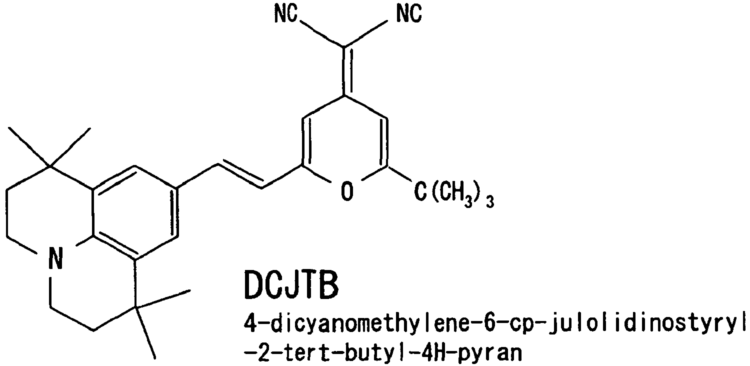

The organic EL element for emitting green light may be properly selected depending on the application; for example, preferable are those having a layer configuration of ITO (positive electrode)/NPD/DPVBi/Alq/Al-Li (negative electrode).

The configuration of the organic EL display may be properly selected depending on the application and may be, for example, a passive-matrix panel or an active-matrix panel as illustrated in "Nikkei Electronics, No. 765, March 13, 2000, pages 55 to 62."

The passive-matrix panel comprises, for example, glass substrate 12, band-like positive electrodes 14 of e.g. indium tin oxide electrodes, organic thin layer 24 for emitting blue light, organic thin layer 26 for emitting green light, organic thin layer 28 for emitting red light, and negative electrodes 22 as shown in FIG. 2. The positive electrodes 14 have a narrow shape, are arranged in parallel with each other on the glass substrate 12. The organic thin layer 24 for emitting blue light, the organic thin layer 26 for emitting blue light, and the organic thin layer 28 for emitting green light are arranged in parallel with one another in turn on the positive electrodes 14 in a direction substantially perpendicular to the positive electrodes 14. The negative electrodes 22 are arranged on the organic thin layer 24 for emitting blue light, the organic thin layer 26 for emitting blue light, and the organic thin layer 28 for emitting red light and have the same shape with these thin layers.

In the passive-matrix panel, positive electrode lines 30 each having plural positive electrodes 14 intersect negative electrode lines 32 each having plural negative electrodes 22 in a substantially perpendicular direction to form a circuit. The organic thin layers 24, 26, and 28 for emitting, blue, green light, and red respectively, are arranged at intersections and serve as pixels. Plural organic EL elements 34 are arranged corresponding to the respective pixels. Upon application of a current by constant-current power supply 36 on one of the positive electrodes 14 in the positive electrode lines 30 and one of the negative electrodes 22 in the negative electrode lines 32 in the passive-matrix panel, the current is applied on an organic EL thin layer at the intersection between the lines to allow the organic EL thin layer at the position to emit light. By controlling light emission of each pixel independently, full-color images can be easily produced.

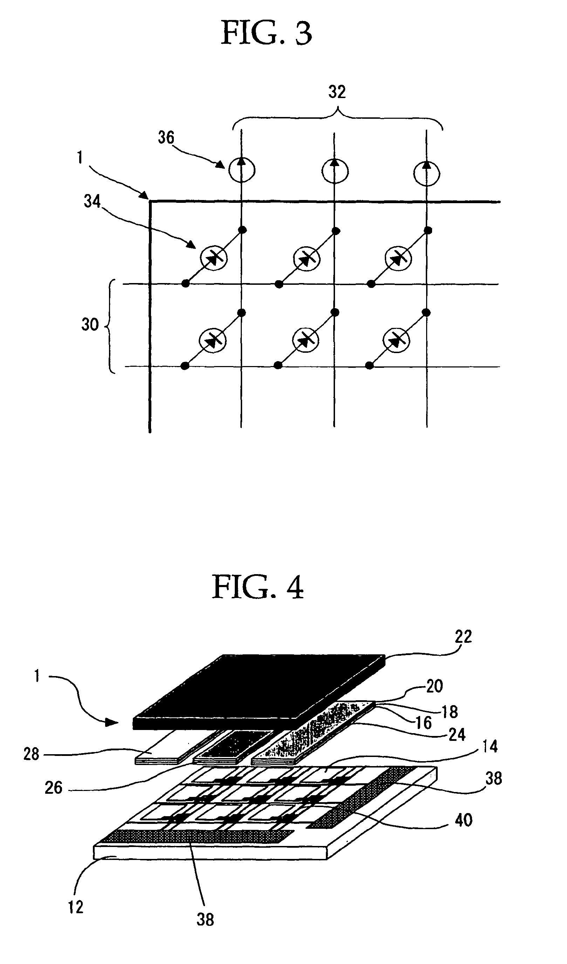

With reference to FIG. 4, the active matrix panel comprises, for example, glass substrate 12, scanning lines, data lines and current supply lines, TFT circuits 40, and positive electrodes 14. The scanning lines, data lines, and current supply lines are arranged on glass substrate 12 as grids in a rectangular arrangement. The TFT circuits 40 are connected typically to the scanning lines constituting the grids and are arranged in each grid. The positive electrodes 14 may be, for example, indium tin oxide electrodes, are capable of being driven by the TFT circuits 40 and are arranged in each grid. Organic thin layer 24 for emitting blue light, organic thin layer 26 for emitting green light, and organic thin layer 28 for emitting red light each has a narrow shape and is arranged in parallel with each other in turn on the positive electrodes 14. Negative electrode 22 is arranged so as to cover organic thin layer 24 for emitting blue light, organic thin layer 26 for emitting green light, and the organic thin layer 28 for emitting red light. The organic thin layer 24 for emitting blue light, the organic thin layer 26 for emitting green light, and the organic thin layer 28 for emitting red light each comprises hole transporting layer 16, light emitting layer 18, and electron transporting layer 20.

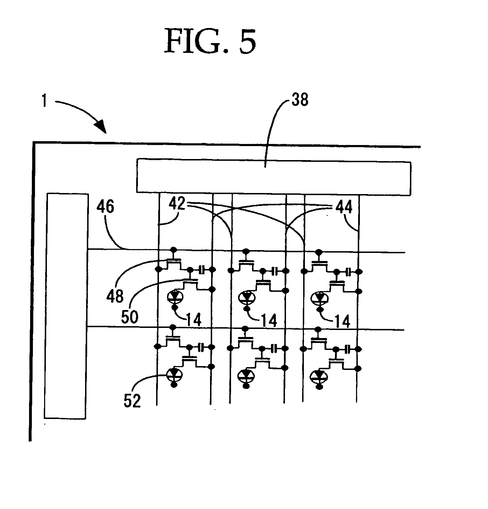

In the active-matrix panel, for example as shown in FIG. 5, scanning lines 46 intersect with data lines 42 and current-supply lines 44 in a perpendicular direction to form grids in a rectangular arrangement. The scanning lines 46 are arranged in parallel with one another. The data lines 42 and current-supply lines 44 are arranged in parallel with one another. Switching TFT 48 and drive TFT 50 are arranged in each grid to form a circuit. The switching TFT 48, and the drive TFT 50 in each grid can be independently derived by the application of a current by drive circuit 38. In each grid, the organic thin film elements 24, 26 and 28 for emitting blue, green, and red lights, respectively serve as pixels. Upon application of a current from the drive circuit 38 to one of the scanning lines 46 arranged in a lateral direction and to the current-supply lines 44 arranged in a vertical direction, switching TFT 48 positioned at the intersection operates to drive the drive TFT 50 to allow organic EL element 52 at the position to emit light. By controlling light emission of each pixel independently, a full-color image can be easily produced.

The organic EL displays according to the present invention are excellent in luminous efficiency, luminance, and color purity, and exhibit stable properties under prolonged usage; therefore, can be properly utilized in a variety of regions such as computers, on-vehicle displays, field displays, household appliances, commercial equipment, household electric equipment, displays for transit, clock displays, calendar displays, luminescent screens and audio equipment.

The present invention will be illustrated more specifically with reference to several examples below, which are not intended to limit the scope of the present invention.

### (Example 1)

### - Synthesis of 1,3,6,8-tetra(4-biphenyl)pyrene -

By reaction of one equivalent of pyrene and four equivalents of bromine, 1,3,6,8-tetrabromopyrene was synthesized in nitrobenzene solvent substantially in accordance with the descriptions in "Annalen der Chemie vol. 531, page 81".

Then, 1,3,6,8-tetrabromopyrene was subjected to a reaction of so-called Suzuki coupling to synthesize 1,3,6,8-tetra(4-biphenyl)pyrene. Namely, 4.4 equivalents of 4-biphenylboronic acid expressed by the following formula, 10 equivalents of sodium carbonate as a solution of 2 mole/liter-water, and 0.12 equivalent of tetrakis(triphenylphosphine)palladium (0) were added to one equivalent of 1,3,6,8-tetrabromopyrene, then the mixture were refluxed for about 3 hours using benzene as a solvent under heating to react these compounds.

Following the reaction, the resulting product was cooled, rinsed several times by water, and the benzene was distilled away. The remaining oily substance was rinsed by methanol, then was recrystallized using a mixed solvent of tetrahydrofuran and methanol thereby to produce a raw reaction product. The raw reaction product was purified by means of vacuum sublimation to obtain 1,3,6,8-tetra(4-biphenylyl)pyrene.

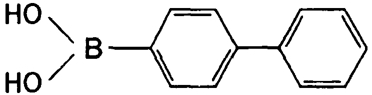

The resulting 1,3,6,8-tetra(4-biphenylyl)pyrene is a compound expressed by the following formula.

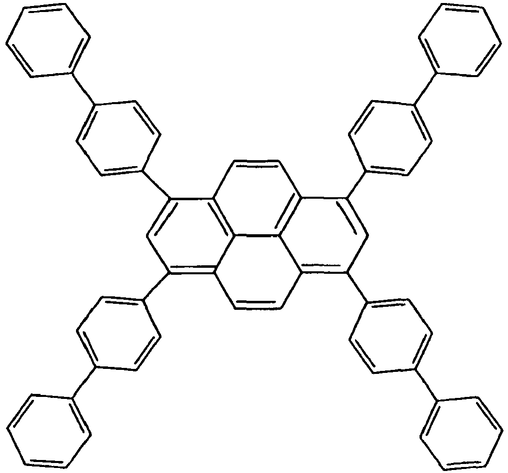

The synthesized 1,3,6,8-tetra(4-biphenylyl)pyrene was subjected to mass spectrometry and infrared (IR) analyses.

### < Result of Mass Spectrometry >

The following result, i.e. m/e = 810, was obtained from the mass spectrometry for the 1,3,6,8-tetra(4-biphenylyl)pyrene, using mass spectrometer Model SX-102A (by JEOL Co.).

### <Result of IR Analysis>

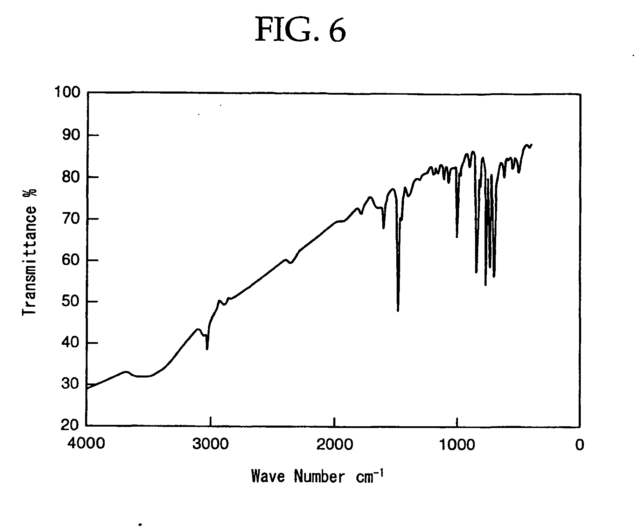

The IR spectrum of the 1,3,6,8-tetra(4-biphenylyl)pyrene according to KBr tablet method is shown in FIG. 6.

### (Example 2)

### - Synthesis of 1,3,6,8-tetra(4-dibenzofuranyl)pyrene -

In the same way as Example 1, 1,3,6,8-tetra(4-dibenzofuranyl)pyrene was synthesized, except for changing the 4-biphenylboronic acid into 4-dibenzofuranboronic acid expressed by the following formula.

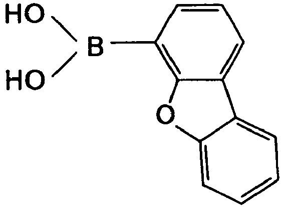

The resulting 1,3,6,8-tetra(4-dibenzofuranyl)pyrene is a compound expressed by the following formula.

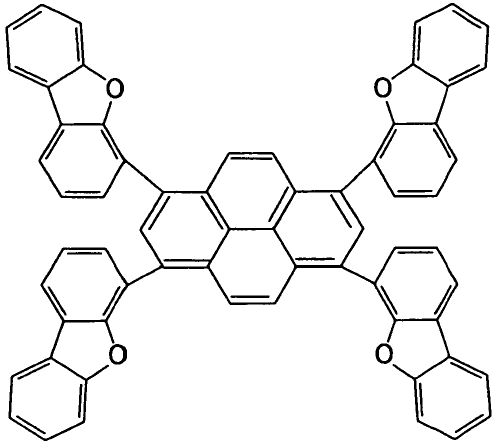

The synthesized 1,3,6,8-tetra(4-dibenzofuranyl)pyrene was subjected to mass spectrometry and IR analyses.

### < Result of Mass Spectrometry >

The following result, i.e. m/e = 866, was obtained from the mass spectrometry for the 1,3,6,8-tetra(4-dibenzofuranyl)pyrene, using mass spectrometer Model SX-102A (by JEOL Co.).

### <Result of IR Analysis>

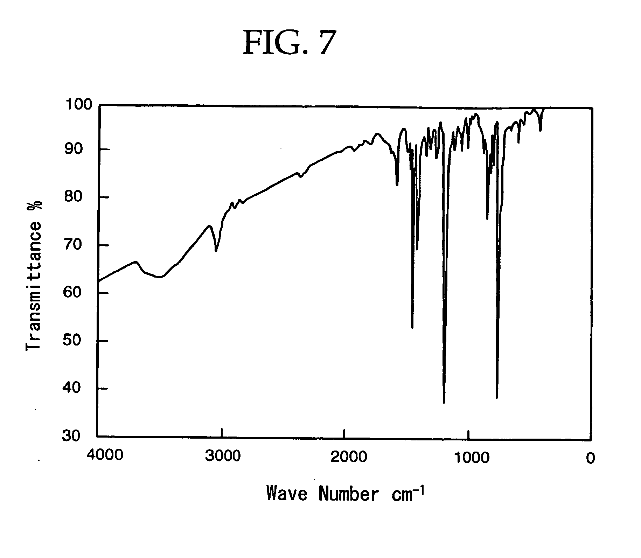

The IR spectrum of the 1,3,6,8-tetra(4-dibenzofuranyl)pyrene according to KBr tablet method is shown in FIG. 7.

### (Example 3)

### - Preparation of Organic EL Element -

A multilayered organic EL element was prepared from 1,3,6,8-tetra(4-biphenyl)pyrene prepared in Example 1 as a light emitting material within a light emitting layer in the following manner. Initially, a glass substrate having an indium tin oxide (ITO) electrode as a positive electrode was subjected to ultrasonic cleaning with water, acetone, and isopropyl alcohol and to UV ozone treatment; thereafter a layer of N,N'-dinaphthyl-N,N'-diphenyl-[1,1'-biphenyl]-4,4'-diamine (NPD) as a hole transporting layer of 50 nm thick was formed on the indium tin oxide electrode using a vacuum vapor deposition apparatus at a vacuum of 1×10⁻⁶ Torr (1.3×10⁻⁴ Pa) and at ambient temperature. Then, a layer of 1,3,6,8-tetra(4-biphenyl)pyrene as a light emitting layer of 30 nm thick was formed by vapor deposition on the hole transporting layer comprising N,N'-dinaphthyl-N,N'-diphenyl-[1,1'-biphenyl]-4,4'-diamine (NPD). Then a layer of aluminum hydroxyquinoline oxybiphenyl complex (BAlq) as an electron transporting layer of 20 nm thick was formed on the light emitting layer by vapor deposition, and a layer of an Al-Li alloy having a Li content of 0.5 percent by mass as a negative electrode was formed to a thickness of 50 nm by vapor deposition on the electron transporting layer comprising the aluminum hydroxyquinoline complex (Alq). Thus, the organic EL element was prepared.

When a voltage was applied to the indium tin oxide (ITO) electrode as the positive electrode and the Al-Li alloy as the negative electrode in the resulting organic EL element, emission of blue light was observed at voltages of 5 V or more, and emission of highly pure blue light having an emission luminance of 1500 cd/m² was observed at a voltage of 10 V.

### (Example 4)

### - Preparation of Organic EL Element -

An organic EL element was prepared in the same way as Example 3, except for forming the light emitting layer by simultaneous vapor deposition of 1,3,6,8-tetra(4-biphenyl)pyrene and N,N'-dinaphthyl-N,N'-diphenyl-[1,1'-biphenyl]-4,4'-diamine (NPD) at a ratio of the vapor deposition rate of the former to that of the latter of 10:90.

When a voltage was applied to the ITO electrode as the positive electrode and the Al-Li alloy as the negative electrode in the resulting organic EL element, emission of blue light was observed at voltages of 4 V or more, and emission of highly pure blue light having an emission luminance of 3860 cd/m² was observed at a voltage of 10 V.

### (Example 5)

### - Preparation of Organic EL Element -

An organic EL element was prepared in the same way as Example 4, except for changing N,N'-dinaphthyl-N,N'-diphenyl-[1,1'-biphenyl]-4,4'-diamine (NPD) as the light emitting material into hydroxyquinoline oxybiphenyl complex (BAlq).

When a voltage was applied to the ITO electrode as the positive electrode and the Al-Li alloy as the negative electrode in the resulting organic EL element, emission of blue light was observed at voltages of 4 V or more, and emission of highly pure blue light having an emission luminance of 3770 cd/m² was observed at a voltage of 10 V.

### (Example 6)

### - Preparation of Organic EL Element -

An organic EL element was prepared in the same way as Example 4, except for changing N,N'-dinaphthyl-N,N'-diphenyl-[1,1'-biphenyl]-4,4'-diamine (NPD) as the light emitting material into 4,4'-bis(9-carbazolyl)-biphenyl (CBP).

When a voltage was applied to the ITO electrode as the positive electrode and the Al-Li alloy as the negative electrode in the resulting organic EL element, emission of blue light was observed at voltages of 4 V or more, and emission of highly pure blue light having an emission luminance of 4790 cd/m² was observed at a voltage of 10 V.

The resulting organic EL element was operated continuously starting from an initial luminance of 150 cd/m²; consequently, the period was 500 hours from the start to the point when the luminance decreased to half of the initial luminance.

### (Example 7)

### - Preparation of Organic EL Element -

An organic EL element was prepared in the same way as Example 6, except 1,3,6,8-tetra(4-biphenylyl)pyrene as the emitting material prepared in Example 1 was changed into 1,3,6,8-tetra(4-dibenzofuranyl)pyrene prepared in Example 2.

When a voltage was applied to the ITO electrode as the positive electrode and the Al-Li alloy as the negative electrode in the resulting organic EL element, emission of blue light was observed at voltages of 5 V or more, and emission of highly pure blue light having an emission luminance of 4500 cd/m² was observed at a voltage of 10 V.

The resulting organic EL element was operated continuously starting from an initial luminance of 150 cd/m²; consequently, the period was 480 hours from the start to the point when the luminance decreased to half of the initial luminance.

### (Comparative Example 1)

### - Preparation of Organic EL Element -

An organic EL element was prepared in the same way as Example 6, except for changing 1,3,6,8-tetra(4-biphenylyl)pyrene was changed into 1,3,6,8-tetraphenylpyrene.

When a voltage was applied to the ITO electrode as the positive electrode and the Al-Li alloy as the negative electrode in the resulting organic EL element, emission of blue light was observed at voltages of 5 V or more, and emission of highly pure blue light having an emission luminance of 680 cd/m² was observed at a voltage of 10 V.

The resulting organic EL element was operated continuously starting from an initial luminance of 150 cd/m²; consequently, the period was 30 hours from the start to the point when the luminance decreased to half of the initial luminance.

### Industrial Applicability

The present invention may provide 1,3,6,8-tetrasubstituted pyrene compounds suited for blue light emitting materials in organic EL elements, organic EL elements that exhibit excellent luminous efficiency, luminance, and color purity in blue light, as well as long lifetime, and organic EL displays that represent high quality and long lifetime.

## Claims

1. An organic electroluminescent element comprising:
a positive electrode,
a negative electrode, and
an organic thin layer arranged between the positive electrode and the negative electrode,
wherein the organic thin layer comprises 1,3,6,8-tetra(4-biphenyl)pyrene expressed by the formula (1-1).

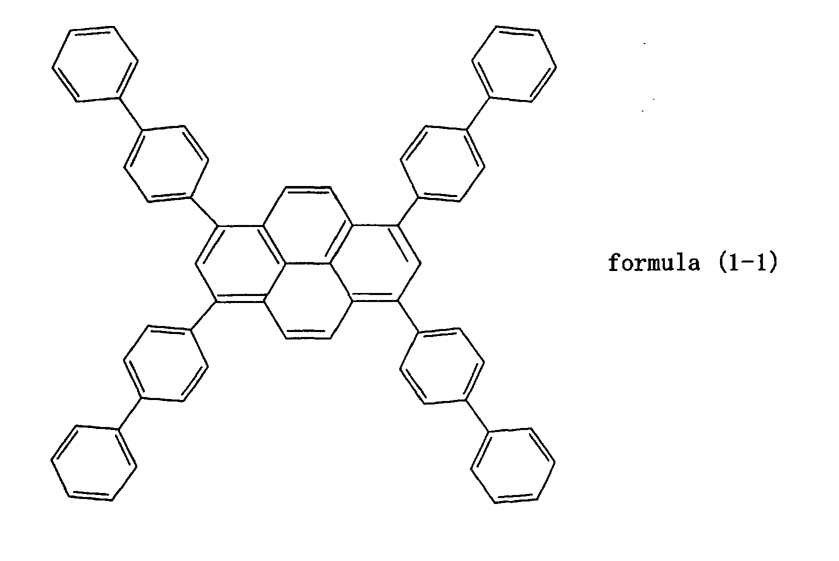

2. An organic electroluminescent element comprising:
a positive electrode,
a negative electrode, and
an organic thin layer arranged between the positive electrode and the negative electrode,
wherein the organic thin layer comprises 1,3,6,8-tetra(4-dibenzofuranyl)pyrene expressed by the formula (1-2).

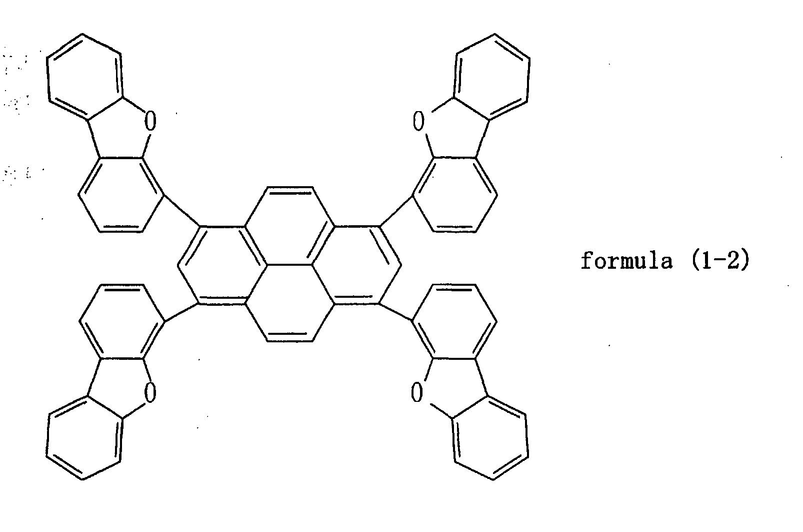

3. The organic electroluminescent element according to one of claims 1 to 2, wherein the organic thin layer comprises a light-emitting electron-transporting layer, and the light-emitting electron-transporting layer contains 1,3,6,8-tetra(4-biphenyl)pyrene or 1,3,6,8-tetra(4-dibenzofuranyl)pyrene as the light emitting material.

4. The organic electroluminescent element according to one of claims 1 to 2, wherein the organic thin layer comprises a light emitting layer interposed between a hole transporting layer and an electron transporting layer, and the light emitting layer contains 1,3,6,8-tetra(4-biphenyl)pyrene or 1,3,6,8-tetra(4-dibenzofuranyl)pyrene as the light emitting material.

5. The organic electroluminescent element according to claim 4, wherein the light emitting layer is formed substantially solely from 1,3,6,8-tetra(4-biphenyl)pyrene or 1,3,6,8-tetra(4-dibenzofuranyl)pyrene.

6. The organic electroluminescent element according to one of claims 4 and 5, wherein the light emitting layer contains an aromatic amine derivative expressed by the formula (7): 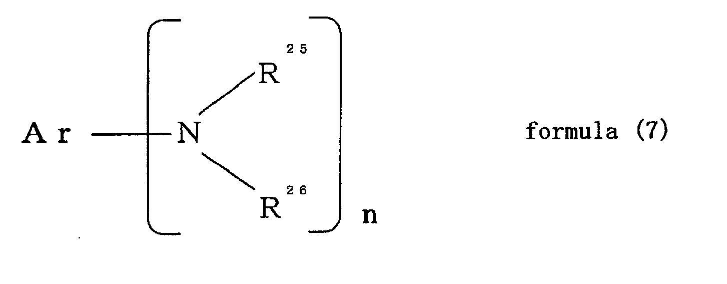 wherein "n" is an integer of 2 or 3; Ar is a divalent or trivalent aromatic or heteroaromatic group; and R²⁵ and R²⁶ may be identical or different each other, and are each a monovalent aromatic or heteroaromatic group.

7. The organic electroluminescent element according to claim 6, wherein the aromatic amine derivative is one of N,N'-dinaphthyl-N,N'-diphenyl-[1,1'-biphenyl]-4,4'- diamine (NPD) expressed by formula (8) and derivatives thereof.

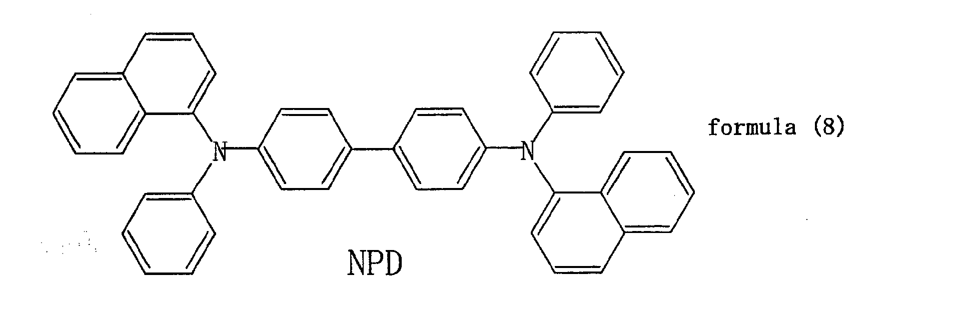

8. The organic electroluminescent element according to one of claims4 to 7, wherein the light emitting layer contains a carbazole derivative expressed by the formula (9): 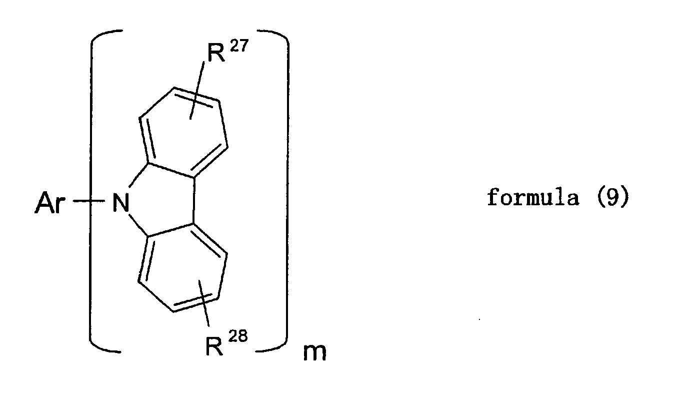 wherein Ar is a divalent or trivalent group containing an aromatic ring or a heteroaromatic group; R²⁷ and R²⁸ are each independently one of hydrogen atom, halogen atoms, alkyl groups, aralkyl groups, alkenyl groups, aryl groups, cyano groups, amino groups, acyl groups, alkoxycarbonyl groups, carboxyl group, alkoxy groups, alkylsulfonyl groups, hydroxy group, amido groups, aryloxy group, aromatic cyclic hydrocarbon groups, heteroaromatic groups, and substituted groups thereof; and "m" is an integer of 2 or 3.

9. The organic electroluminescent element according to claim 8, wherein the carbazole derivative is one of 4,4'-bis(9-carbazolyl)-biphenyl (CBP) expressed by formula (10) and derivatives thereof.

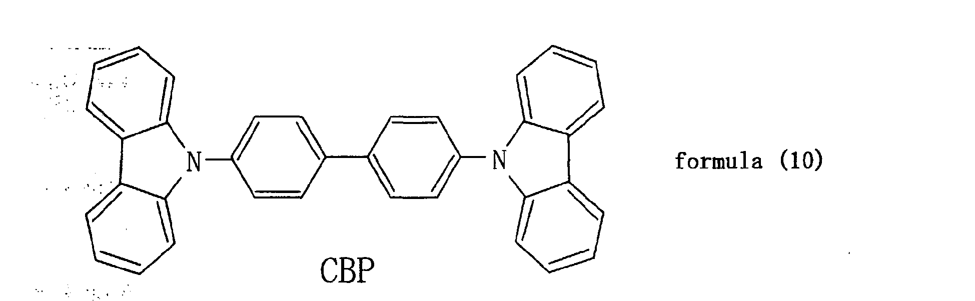

10. The organic electroluminescent element according one of to claims 4 to 7, wherein the light emitting layer contains a hydroxyquinoline oxyaryl complex expressed by the formula (11): 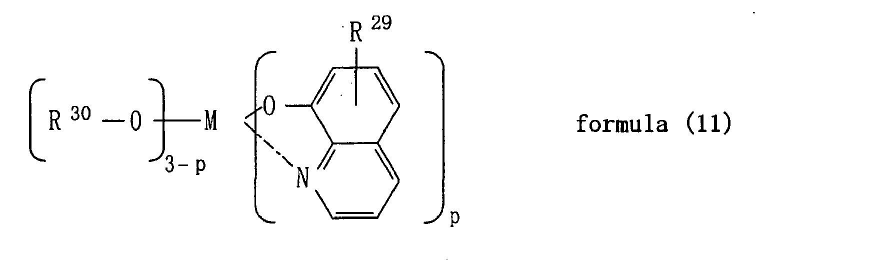 wherein M is a trivalent metal atom; R²⁹ is a hydrogen atom or an alkyl group; R³⁰ is a hydrogen atom or an aryl group; and "p" is an integer of 1 or 2.

11. The organic electroluminescent element according to claim 10, wherein the hydroxyquinoline oxyaryl complex is an aluminum hydroxyquinoline oxybiphenyl complex (BAlq) expressed by formula (12).

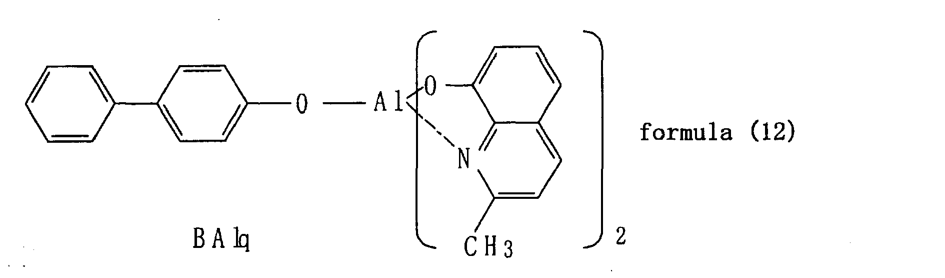

12. The organic electroluminescent element according to one of claims 4 to 11, wherein the electron transporting material in the electron transporting layer is 2,9-dimethyl-4,7-diphenyl-1,10-phenanthroline (BCP) expressed by the formula (18).

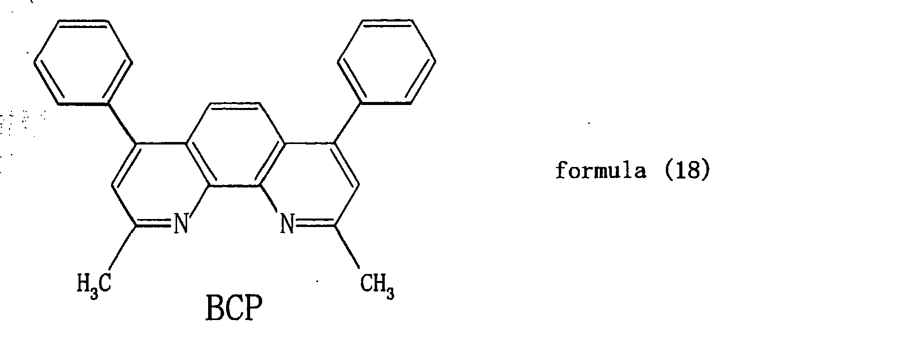

13. The organic electroluminescent element according to one of claims 1 to 12, wherein the organic electroluminescent element is a blue light emitting element.

14. 1,3,6,8-tetra(4-biphenyl)pyrene expressed by the formula (1-1).

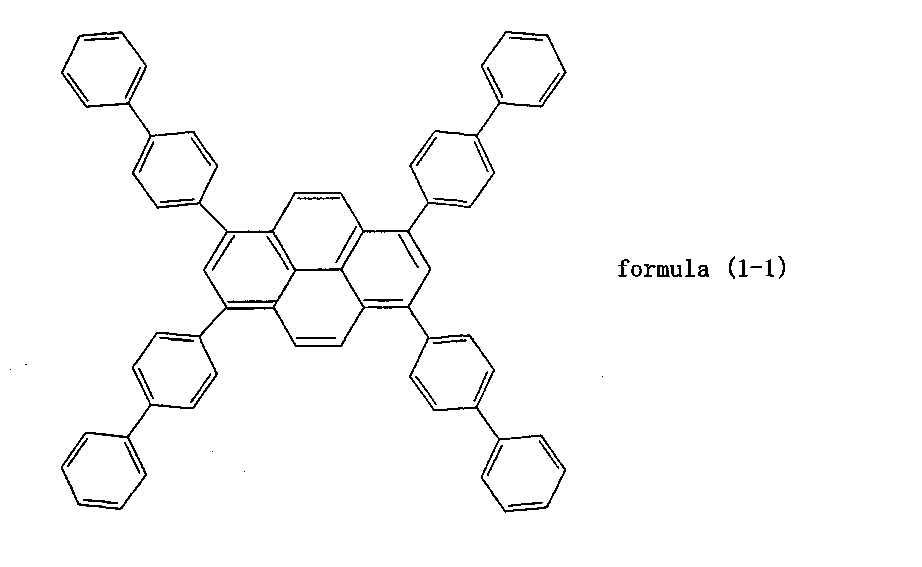

15. 1,3,6,8-tetra(4-dibenzofuranyl)pyrene expressed by the formula (1-2).

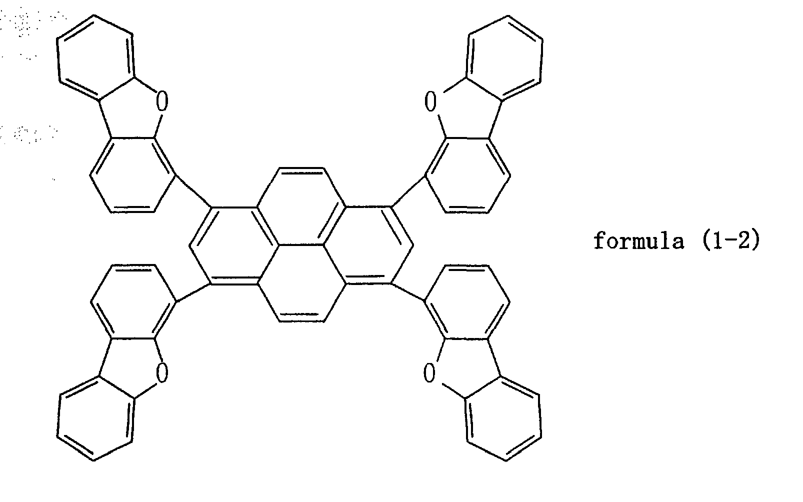

16. A 1,3,6,8-tetrasubstituted pyrene compound selected from 1,3,6,8-tetra(4-biphenyl)pyrene and 1,3,6,8-tetra(4-dibenzofuranyl)pyrene, wherein the 1,3,6,8-tetrasubstituted pyrene compound is utilized as a light emitting material in an organic electroluminescent element.

17. An organic electroluminescent display, wherein the organic electroluminescent display is equipped with the organic electroluminescent element according to one of claims 1 to 13.

18. The organic electroluminescent display according to claim 17, wherein the organic electroluminescent display is one of passive matrix panels and active matrix panels.

## Patentansprüche

1. Organisches Elektrolumineszenz-Element, umfassend:
eine positive Elektrode,
eine negative Elektrode und
eine organische Dünnschicht zwischen der positiven Elektrode und der negativen Elektrode,
wobei die organische Dünnschicht 1,3,6,8-Tetra(4-biphenyl)pyren mit der Formel (1-1) umfasst:

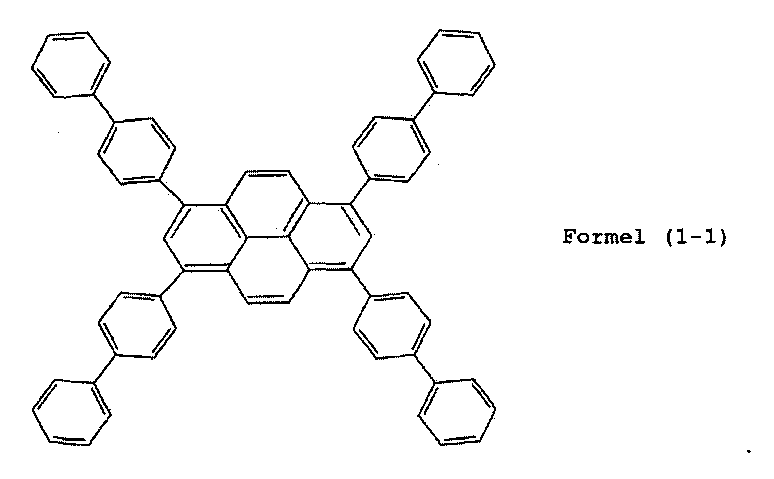

2. Organisches Elektrolumineszenz-Element, umfassend:
eine positive Elektrode,
eine negative Elektrode, und
eine organische Dünnschicht zwischen der positiven Elektrode und der negativen Elektrode,
wobei die organische Dünnschicht 1,3,6,8-Tetra(4-dibenzofuranyl)pyren mit der Formel (1-2) umfasst:

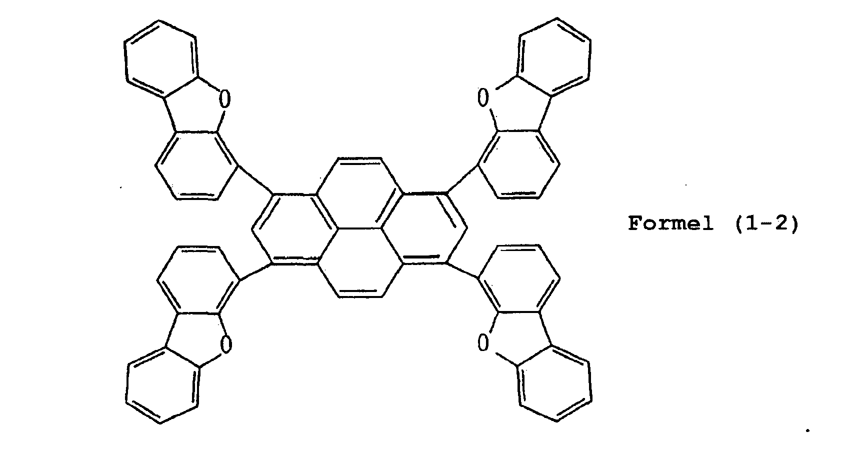

3. Organisches Elektrolumineszenz-Element gemäß einem der Ansprüche 1 bis 2, wobei die organische Dünnschicht eine lichtemittierende Elektronen-transportierende Schicht umfasst, und die lichtemittierende, Elektronen-transportierende Schicht 1,3,6,8-Tetra(4-biphenyl)pyren oder 1,3,6,8-Tetra(4-dibenzofuranyl)pyren als lichtemittierendes Material enthält.

4. Organisches Elektrolumineszenz-Element gemäß einem der Ansprüche 1 bis 2, wobei die organische Dünnschicht eine lichtemittierende Schicht umfasst, die zwischen einer Loch-transportierenden Schicht und einer Elektronen-transportierenden Schicht eingefügt ist, und die lichtemittierende Schicht 1,3,6,8-Tetra(4-biphenyl)pyren oder 1,3,6,8-Tetra(4-dibenzofuranyl)pyren als lichtemittierendes Material enthält.

5. Organisches Elektrolumineszenz-Element gemäß Anspruch 4, wobei die lichtemittierende Schicht im Wesentlichen ausschließlich aus 1,3,6,8-Tetra(4-biphenyl)pyren oder 1,3,6,8-Tetra(4-dibenzofuranyl)pyren gebildet wird.

6. Organisches Elektrolumineszenz-Element gemäß einem der Ansprüche 4 und 5, wobei die lichtemittierende Schicht ein aromatisches Amin-Derivat mit der Formel (7) enthält: 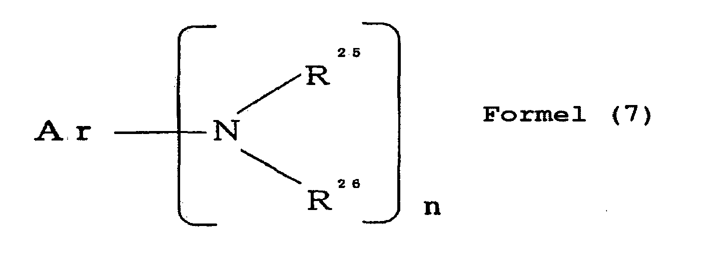 wobei "n" eine ganze Zahl von 2 oder 3 ist, Ar eine divalente oder trivalente, aromatische oder heteroaromatische Gruppe ist; und R²⁵ und R²⁶ gleich oder verschieden voneinander sein können und jeweils eine monovalente, aromatische oder heteroaromatische Gruppe sind.

7. Organisches Elektrolumineszenz-Element gemäß Anspruch 6, wobei das aromatische Amin-Derivat ein Vertreter von N,N'-Dinaphthyl-N,N'-diphenyl-[1,1'-biphenyl]-4,4'-diamin (NPD) mit der Formel (8) und Derivaten hiervon ist:

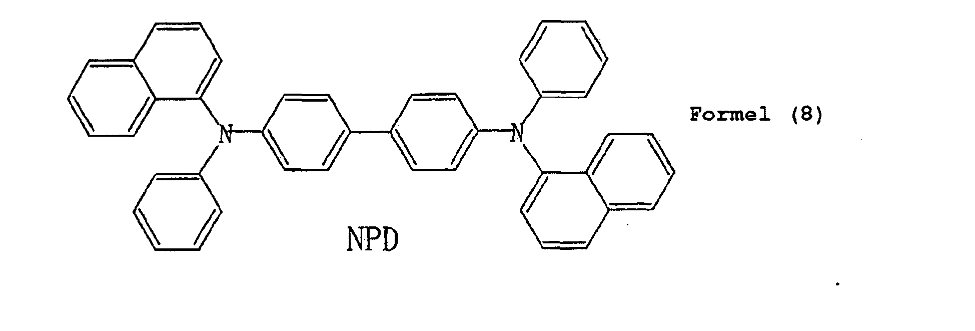

8. Organisches Elektrolumineszenz-Element gemäß einem der Ansprüche 4 bis 7, wobei die lichtemittierende Schicht ein Carbazol-Derivat gemäß der Formel (9) enthält: 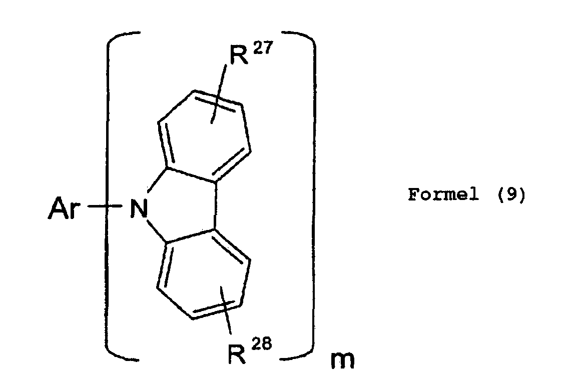 wobei Ar eine divalente oder trivalente Gruppe ist, die einen aromatischen Ring oder eine heteroaromatische Gruppe enthält; R²⁷ und R²⁸ jeweils unabhängig voneinander ein Wasserstoffatom, Halogenatome, Alkylgruppen, Aralkylgruppen, Alkenylgruppen, Arylgruppen, Cyanogruppen, Aminogruppen, Acylgruppen, Alkoxycarbonylgruppen, Carboxylgruppen, Alkoxygruppen, Alkylsulfonylgruppen, Hydroxygruppe, Amidogruppen, Aryloxygruppe, aromatische zyklische Kohlenwasserstoffgruppen, heteroaromatische Gruppen, und substituierte Gruppen hiervon sind; und "m" eine ganze Zahl von 2 oder 3 ist.

9. Organisches Elektrolumineszenz-Element gemäß Anspruch 8, wobei das Carbazol-Derivat ein Vertreter von 4,4'-Bis(9-carbazolyl)-biphenyl (CBP) mit der Formel (10) und Derivaten hiervon ist:

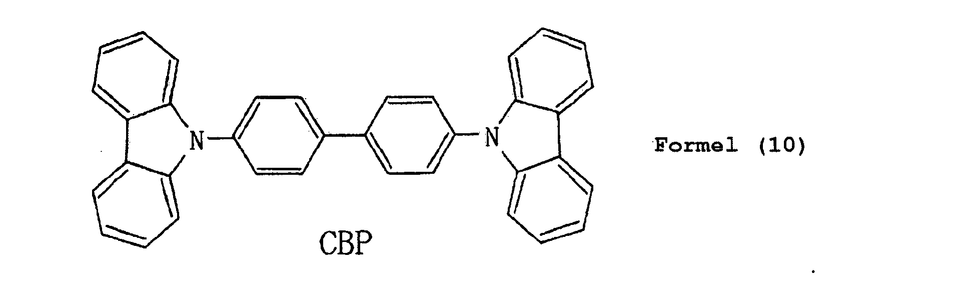

10. Organisches Elektrolumineszenz-Element gemäß einem der Ansprüche 4 bis 7, wobei die lichtemittierende Schicht einen Hydroxychinolinoxyaryl-Komplex mit der Formel (11) enthält: 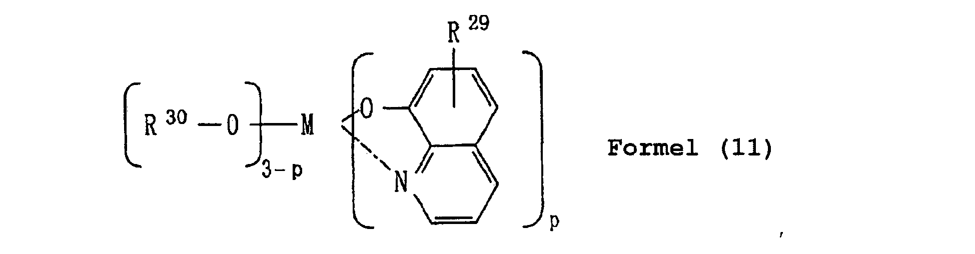 wobei M ein trivalentes Metallatom ist; R²⁹ ein Wasserstoffatom oder eine Alkylgruppe ist; R³⁰ ein Wasserstoffatom oder eine Arylgruppe ist; und "p" eine ganze Zahl von 1 oder 2 ist.

11. Organisches Elektrolumineszenz-Element gemäß Anspruch 10, wobei der Hydroxychinolinoxyaryl-Komplex ein Aluminiumhydroxychinolinoxybiphenyl-Komplex (BAlq) mit der Formel (12) ist:

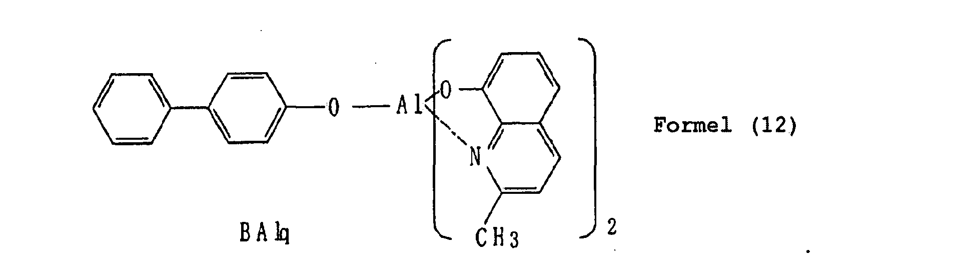

12. Organisches Elektrolumineszenz-Element gemäß einem der Ansprüche 4 bis 11, wobei das Elektronen-transportierende Material in der Elektronen-transportierenden Schicht 2,9-Dimethyl-4,7-diphenyl-1,10-phenanthrolin (BCP) mit der Formel (18) ist:

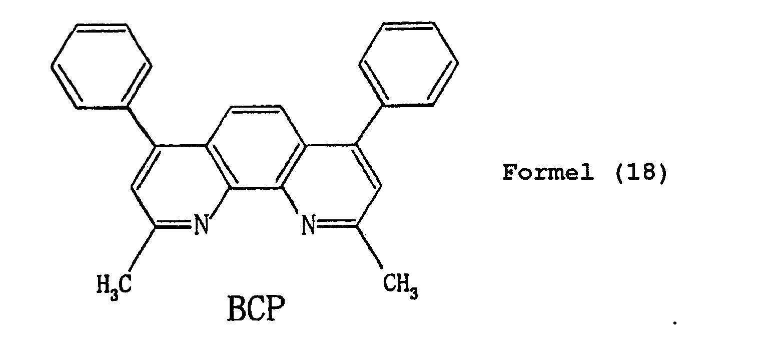

13. Organisches Elektrolumineszenz-Element gemäß einem der Ansprüche 1 bis 12, wobei das organische Elektrolumineszenz-Element ein Element ist, das blaues Licht emittiert.

14. 1,3,6,8-Tetra(4-biphenyl)pyren mit der Formel (1-1):

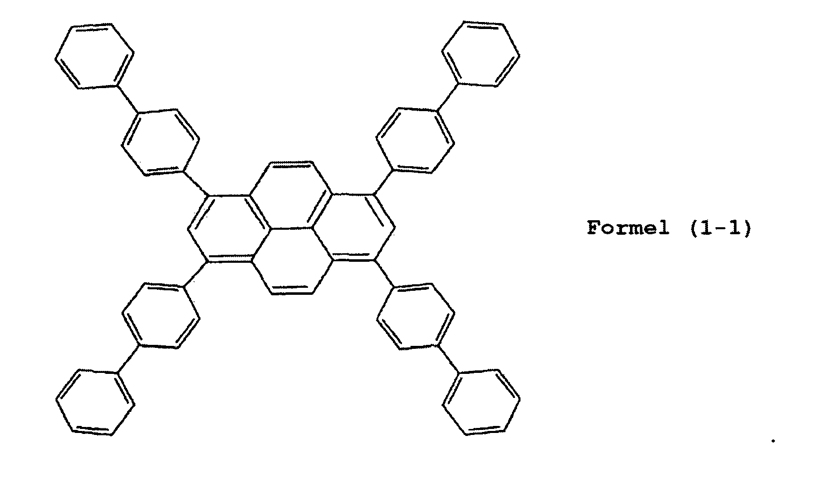

15. 1,3,6,8-Tetra(4-dibenzofuranyl)pyren mit der Formel (1-2)

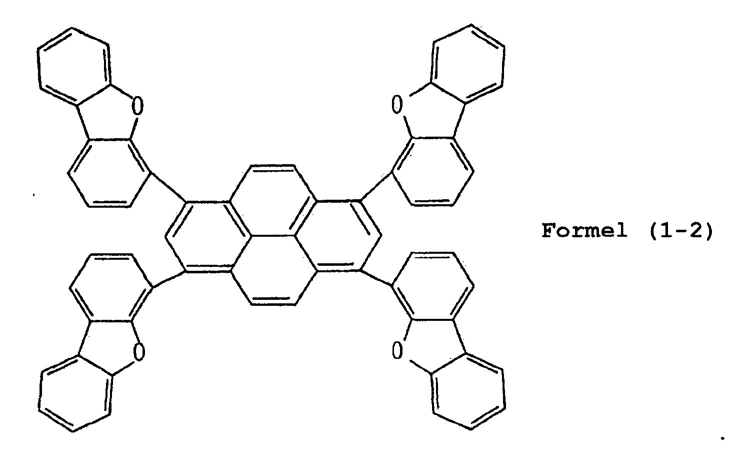

16. 1,3,6,8-Tetra-substituierte Pyrenverbindung, ausgewählt aus 1,3,6,8-Tetra(4-biphenyl)pyren und 1,3,6,8-Tetra(4-dibenzofuranyl)pyren, wobei die 1,3,6,8-tetrasubstituierte Pyrenverbindung als ein lichtemittierendes Material in einem organischen Elektrolumineszenz-Element eingesetzt wird.

17. Organische Elektrolumineszenz-Anzeige, wobei die organische Elektrolumineszenz-Anzeige mit dem organischen Elektrolumineszenz-Element gemäß einem der Ansprüche 1 bis 13 ausgestattet ist.

18. Organische Elektrolumineszenz-Anzeige gemäß Anspruch 17, wobei die organische Elektrolumineszenz-Anzeige ein passives Matrix-Panel oder ein aktives Matrix-Panel ist.

## Revendications

1. Élément électroluminescent organique comprenant :
une électrode positive,
une électrode négative et
une couche mince organique disposée entre l'électrode positive et l'électrode négative,
dans lequel la couche mince organique comprend le 1,3,6,8-tétra(4-biphényl)pyrène exprimé par la formule (1-1)

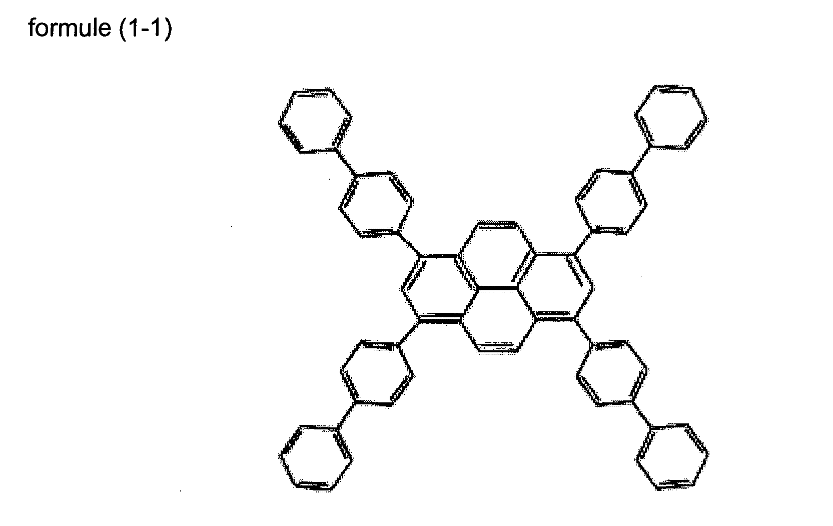

2. Élément électroluminescent organique comprenant :
une électrode positive,
une électrode négative et
une couche mince organique disposée entre l'électrode positive et l'électrode négative,
dans lequel la couche mince organique comprend le 1,3,6,8-tétra(4-dibenzofuranyl)pyrène exprimé par la formule (1-2).

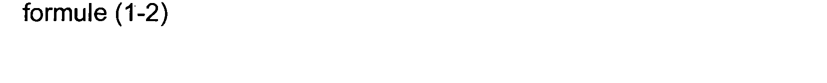

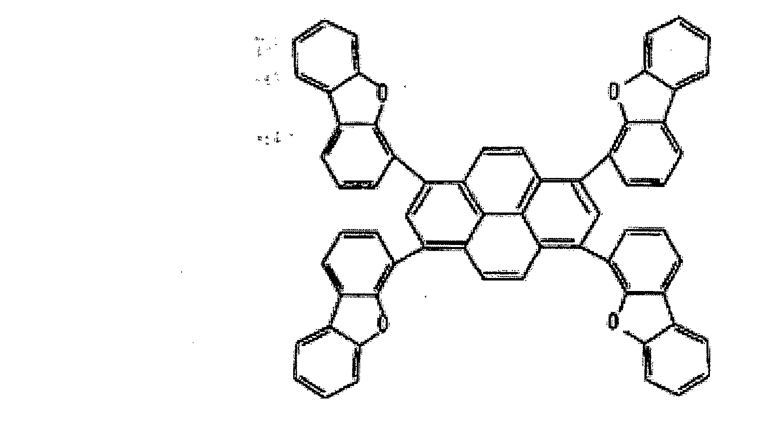

3. Élément électroluminescent organique selon l'une des revendications 1 à 2, dans lequel la couche mince organique comprend une couche de transport d'électrons émettrice de lumière, et la couche de transport d'électrons émettrice de lumière contient le 1,3,6,8-tétra(4-biphényl)pyrène ou le 1,3,6,8-tétra(4-dibenzofuranyl)pyrène comme matériau émetteur de lumière.

4. Élément électroluminescent organique selon l'une des revendications 1 à 2, dans lequel la couche mince organique comprend une couche émettrice de lumière interposée entre une couche de transport de trous et une couche de transport d'électrons et la couche émettrice de lumière contient du 1,3,6,8-tétra(4-biphényl)pyrène ou du 1,3,6,8-tétra(4-dibenzofuranyl)pyrène comme matériau émetteur de lumière.

5. Élément électroluminescent organique selon la revendication 4, dans lequel la couche émettrice de lumière est formée essentiellement seulement de 1,3,6,8-tétra(4-biphénylpyrène) ou de 1,3,6,8-tétra(4-dibenzofuranyl)pyrène.

6. Élément électroluminescent organique selon une des revendications 4 et 5, dans lequel la couche émettrice de lumière contient un dérivé d'amine aromatique exprimé par la formule 7 : 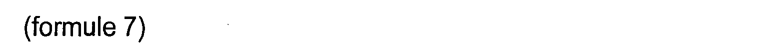 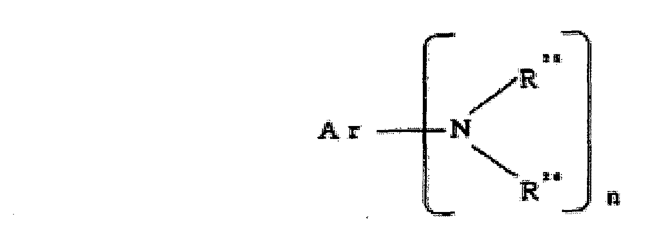 dans laquelle « n » est un nombre entier de 2 ou 3 ; Ar est un groupe aromatique ou hétéroaromatique divalent ou trivalent ; et R²⁵ et R²⁶ peuvent être identiques ou différents l'un de l'autre, et sont chacun un groupe aromatique ou hétéroaromatique monovalent.

7. Élément électroluminescent organique selon la revendication 6, dans lequel le dérivé d'amine aromatique est une parmi la N,N'--dinaphtyl-N,N'-diphényl-[1,1'-biphényl]-4,4'-diamine (NPD) exprimée par la formule 8 et les dérivés associés.

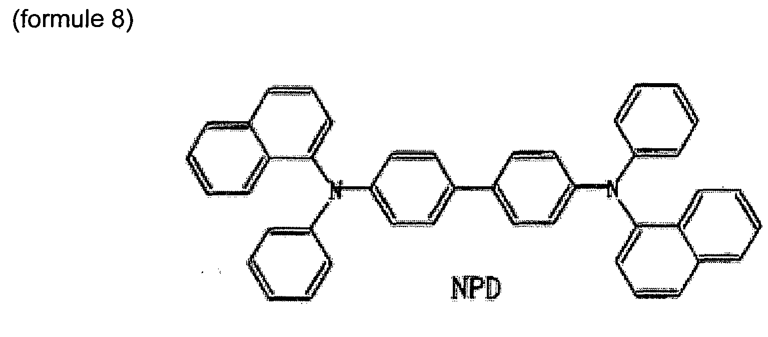

8. Élément électroluminescent organique selon l'une des revendications 4 à 7, dans lequel la couche émettrice de lumière contient un dérivé de carbazole exprimé par la formule (9) : 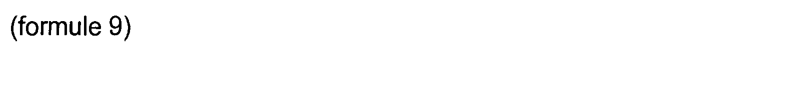 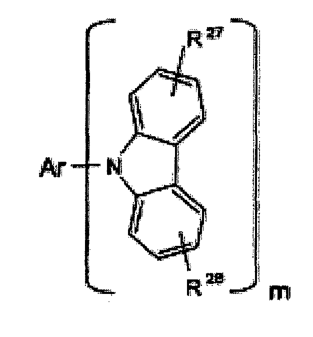 dans laquelle Ar est un groupe divalent ou trivalent contenant un cycle aromatique ou un groupe hétéroaromatique ; R²⁷ et R²⁸ sont chacun indépendamment un parmi un atome d'hydrogène, des atomes d'halogène, des groupes alkyle, des groupes aralkyle, des groupes alcényle, des groupes aryle, des groupes cyano, des groupes amino, des groupes acyle, des groupes alcoxycarbonyle, un groupe carboxyle, des groupes alcoxy, des groupes alkylsulfonyle, un groupe hydroxy, des groupes amido, un groupe aryloxy, des groupes hydrocarbonés cycliques aromatiques, des groupes hétéroaromatiques et les groupes substitués de ceux-ci ; et « m » est un nombre entier de 2 ou 3.

9. Élément électroluminescent organique selon la revendication 8, dans lequel le dérivé de carbazole est un parmi le 4,4'-bis(9-carbazolyl)-biphényle (CBP) exprimé par la formule (10) et les dérivés associés.

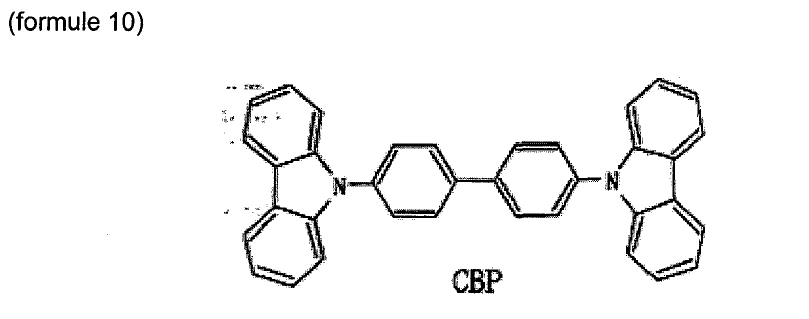

10. Élément électroluminescent organique selon l'une des revendications 4 à 7, dans lequel la couche émettrice de lumière contient un complexe hydroxyquinoline oxyaryle exprimé par la formule (11) : 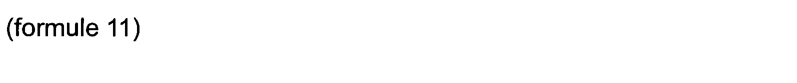 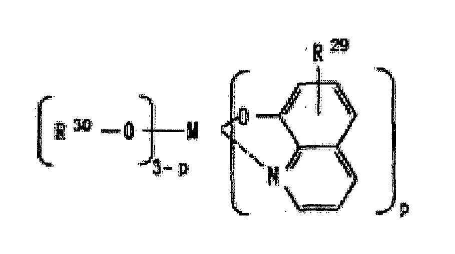 dans laquelle M est un atome métallique trivalent ; R²⁹ est un atome d'hydrogène ou un groupe alkyle ; R³⁰ est un atome d'hydrogène ou un groupe aryle ; et « p » est un nombre entier de 1 ou 2.

11. Élément électroluminescent organique selon la revendication 10, dans lequel le complexe hydroxyquinoline oxyaryle est un complexe hydroxyquinoline oxybiphényle d'aluminium (BAlq) exprimé par la formule 12.

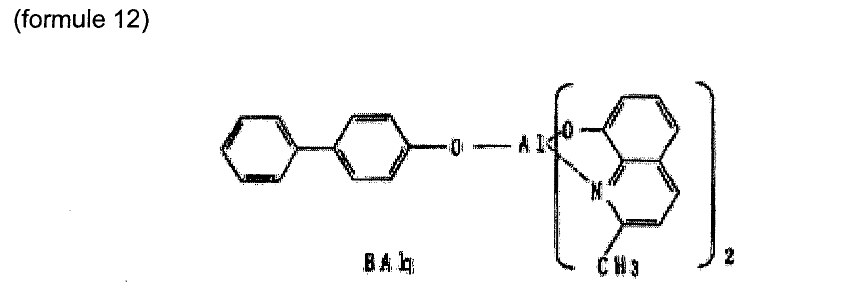

12. Élément électroluminescent organique selon l'une des revendications 4 à 11, dans lequel le matériau transporteur d'électrons dans la couche de transport d'électrons est la 2,9-diméthyl-4,7-diphényl-1,10-phénanthroline (BCP) exprimée par la formule 18.

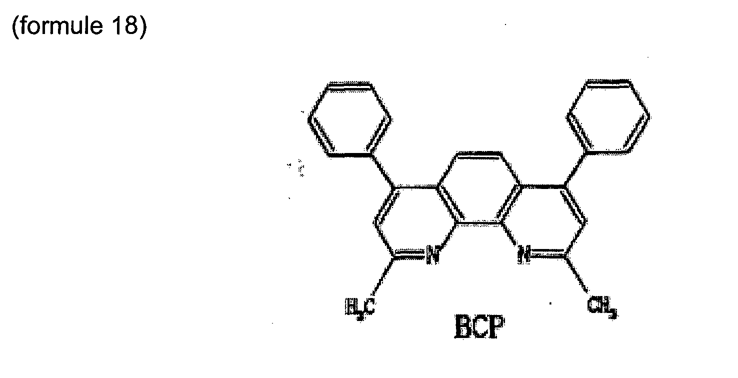

13. Élément électroluminescent organique selon l'une des revendications 1 à 12, dans lequel l'élément électroluminescent organique est un élément émetteur de lumière bleue.

14. 1,3,6,8-tétra(4-biphényl)pyrène exprimé par la formule (1-1).

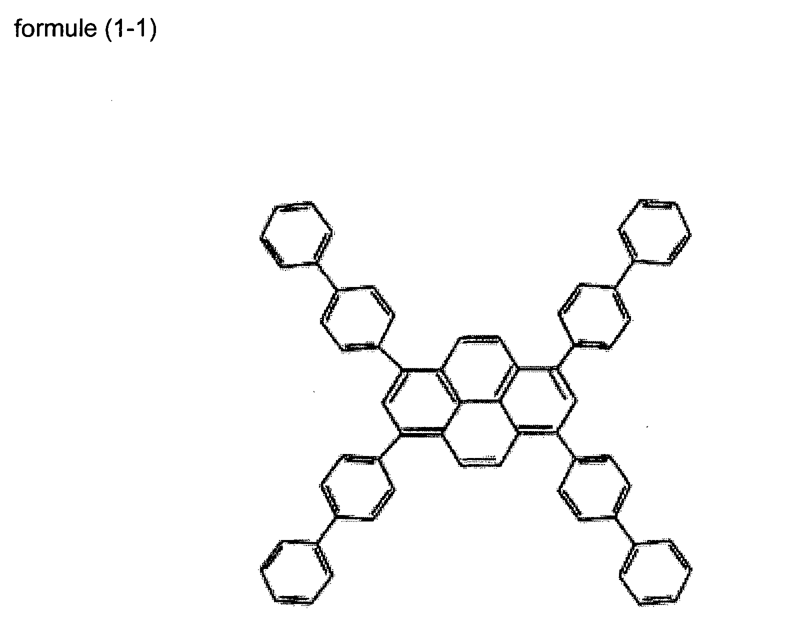

15. 1,3,6,8-tétra(4-dibenzofuranyl)pyrène exprimé par la formule (1-2).

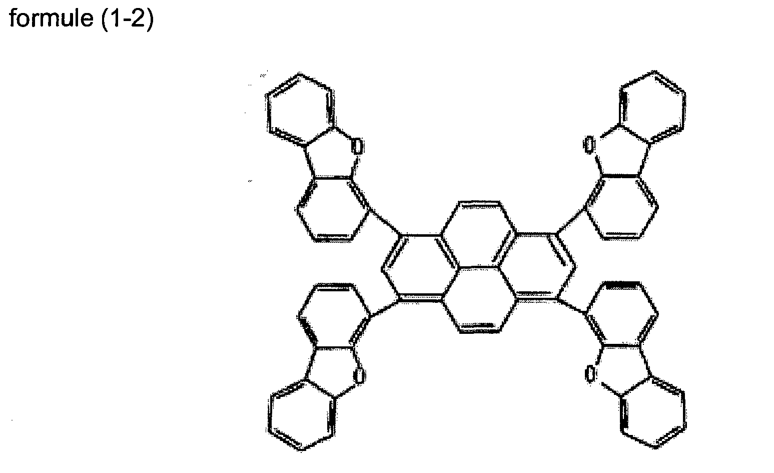

16. Composé pyrène 1,3,6,8-tétrasubstitué choisi parmi le 1,3,6,8-tétra(4-biphényl)pyrène et le 1,3,6,8-tétra(4-dibenzofuranyl)pyrène, dans lequel le composé pyrène 1,3,6,8-tétrasubstitué est utilisé comme matériau émetteur de lumière dans un élément électroluminescent organique.

17. Affichage électroluminescent organique, dans lequel l'affichage électroluminescent organique est équipé de l'élément électroluminescent organique selon l'une des revendications 1 à 13.

18. Affichage électroluminescent organique selon la revendication 17, dans lequel l'affichage électroluminescent organique est un parmi les panneaux à matrice passive et les panneaux à matrice active.
